(19) Europäisches Patentamt
European Patent Office
Office européen des brevets

(11) Veröffentlichungsnummer: **0 335 144 A1**

# (12) EUROPÄISCHE PATENTANMELDUNG

(21) Anmeldenummer: 89104145.1

(22) Anmeldetag: 09.03.89

(51) Int. Cl.[4]: C07D 249/08 , A61K 31/41 , C07D 409/14 , C07D 401/12 , C07D 403/12 , C07D 413/14 , C07D 401/14 , C07D 405/14

(30) Priorität: 12.03.88 DE 3808283

(43) Veröffentlichungstag der Anmeldung: 04.10.89 Patentblatt 89/40

(84) Benannte Vertragsstaaten: AT BE CH DE ES FR GB GR IT LI LU NL SE

(71) Anmelder: **BOEHRINGER INGELHEIM KG**
Postfach 200
D-6507 Ingelheim am Rhein(DE)

(84) AT BE CH DE ES FR GB GR IT LI LU NL SE

Anmelder: **BOEHRINGER INGELHEIM INTERNATIONAL G.M.B.H.**
Postfach 20
D-6507 Ingelheim am Rhein(DE)

(84) GB

(72) Erfinder: **Schromm, Kurt, Dr. Chem.**
In der Dörrwiese 35
D-6507 Ingelheim(DE)
Erfinder: **Walther, Gerhard, Dr. Chem.**
Pfarrer-Heberer-Strasse 37
D-6530 Bingen(DE)
Erfinder: **Heuer, Hubert, Dr.**
Im Alten Weg 29
D-6500 Mainz 32(DE)
Erfinder: **Muacevic, Gojko, Dr.**
In der Dörrwiese 13
D-6507 Ingelheim(DE)
Erfinder: **Birke, Franz, Dr.**
Albrecht-Dürer-Strasse 23
D-6507 Ingelheim(DE)

## (54) Neue 3,4,5-substituierte 4H-1,2,4-Triazole, ihre Herstellung und Verwendung.

(57) Die neuen Verbindungen der Formel

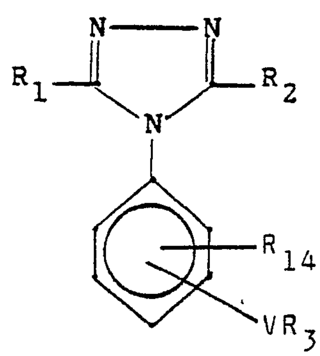

(I)

($R_1$, $R_2$, $R_3$, $R_{14}$ und V sind in der Beschreibung definiert), können nach üblichen Methoden hergestellt und als Arzneistoffe mit insbesondere PAF-antagonistischer Wirkung verwendet werden.

EP 0 335 144 A1

## Neue 3,4,5-substituierte 4H-1,2,4-Triazole, ihre Herstellung und Verwendung

Die Erfindung betrifft neue 3,4,5-substituierte 4-H-1,2,4-Triazole, ihre Herstellung nach an sich bekannten Verfahren und ihre Verwendung als Arzneistoffe.

Die neuen Verbindungen entsprechen der Formel

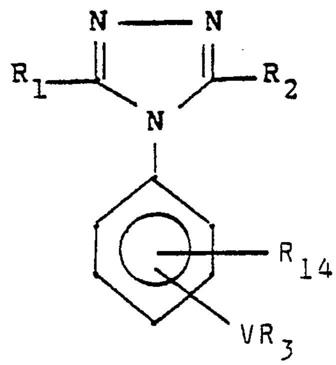

(I)

Darin haben $R_1$, $R_2$, $R_3$, $R_{14}$ und V folgende Bedeutungen:

$R_1$:

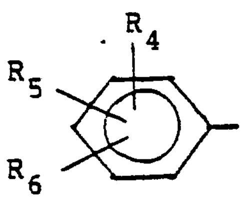

,

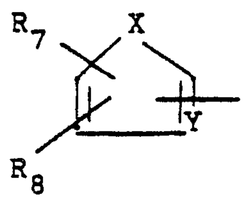

,

(II) (III)

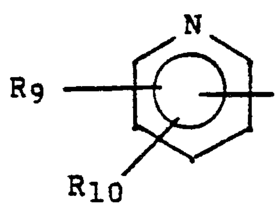

(IV)

wobei

$R_4$, $R_5$ für H, $R_{11}$, $OR_{11}$, Halogen, $CF_3$, Aryl, $N(R_{11})_2$, gemeinsam auch für einen ankondensierten $C_5$-$C_7$-Cycloalken-, einen ankondensierten Benzolring oder eine der an benachbarte C-Atome des Benzolrings gebundene Gruppen -N=CH-NH-, -N=CH-CH=CH-, $-O-CH_2-O-$, $-O-CH_2-CH_2-O-$, -NH-CO-NH-und -N=CH-CH=N-,

$R_6$ für H, $R_{11}$ oder $OR_{11}$ oder Aryl;

$R_7$ für H, $R_{11}$, Halogen oder Aryl;

$R_8$ für H, $R_{11}$ oder Halogen, $R_7$ und $R_8$ gemeinsam auch für einen ankondensierten $C_5$-$C_7$-Cycloalken- oder Benzolring,

$R_9$, $R_{10}$ für H, $R_{11}$, $OR_{11}$, $N(R_{11})_2$, gemeinsam auch für einen ankondensierten Benzolring;

X für S, O, NH oder N($C_1$-$C_4$-Alkyl);

Y für CH, N;

$R_{11}$ für $C_1$-$C_4$-Alkyl, und, wenn neben $R_{11}$ bzw. einer $R_{11}$ enthaltenden Gruppe die Reste II, III bzw. IV keine weiteren Substituenten aufweisen, auch $C_5$-$C_{12}$-Alkyl oder -Alkoxy stehen;

$R_2$: H, $C_1$-$C_4$-Alkyl, $CF_3$, $C_1$-$C_4$-Alkoxy, $CH_2$-O-($C_1$-$C_4$-Alkyl), $C_3$-$C_6$-Cycloalkyl;

$R_3$ ($C_1$-$C_6$-Alkylen)-Het, wobei Het

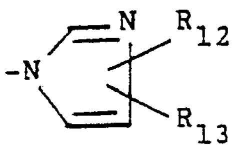 , 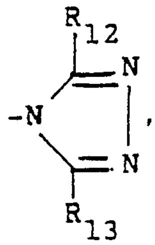 , 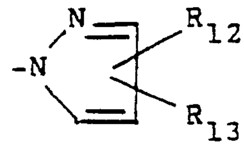

(V) (VI) (VII)

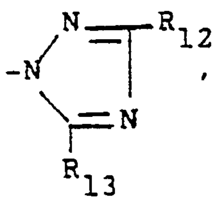 , 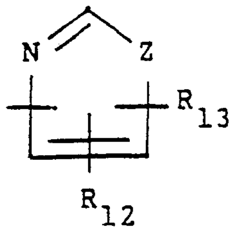 , 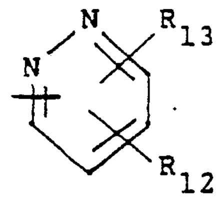 .

(VIII) (IX) (X)

Z: S, N-$R_{12}$, CH=CH, N=CH bzw. CH=N;
$R_{12}$: H, $C_1$-$C_4$-Alkyl oder Aryl;
$R_{13}$: H, $C_1$-$C_4$-Alkyl, $R_{12}$ und $R_{13}$ gemeinsam gegebenenfalls einen ankondensierten Benzolring bedeuten;
$R_{14}$: H, $C_1$-$C_4$-Alkyl, $C_1$-$C_4$-Alkoxy oder Halogen; ($R_{14}$ kann jeweils auch doppelt vorhanden sein);
V: O oder S;
sowie die Salze dieser Verbindungen mit Säuren.

Die Kohlenwasserstoffketten in den definierten Resten können geradkettig oder verzweigt sein. "Halogen" bedeutet Fluor, Chlor, Brom und auch Jod, "Aryl" vor allem Phenyl und Naphthyl. Soweit Mehrfachsubstitution in den heterocyclischen und aromatischen Resten möglich ist, können die Substituenten gleich oder verschieden sein. Dabei können bis zu drei Alkyl- oder Alkoxygruppen vorliegen, insbesondere im Fall von $C_1$-$C_2$-Alkyl- bzw. Alkoxy, während Aryl, $CF_3$, $N(R_{11})_2$ höchstens zweimal, bevorzugt aber nur einmal vorliegt. Die Gruppe $N(R_{11})_2$ kann gleiche oder verschiedene Reste $R_{11}$ enthalten. Die Gruppe $VR_3$ befindet sich bevorzugt in para-Stellung zur Triazolylgruppe. Im Rahmen der vorstehenden Definitionen sind die folgenden Substituentenbedeutungen hervorzuheben:

$R_1$: die Gruppen II und III,
wobei
$R_4$, $R_5$ und $R_6$ H, $C_1$-$C_4$-Alkyl, $C_1$-$C_4$-Alkoxy, Halogen, $R_4$ auch $C_5$-$C_{12}$-Alkyl oder -Alkoxy, wenn $R_5$ und $R_6$ H sind,
$R_4$ und $R_5$ gemeinsam auch ankondensiertes $C_5$-$C_7$-Cycloalken, Methylendioxy, Ethylendioxy sowie, falls $R_6$ H ist, auch einen ankondensierten Benzolring bedeuten;
X S, O, NH, N($C_1$-$C_4$-Alkyl);
Y N, wenn X NH oder N-($C_1$-$C_4$-Alkyl) ist, CH, wenn X S oder O bedeutet;
$R_7$, $R_8$ H, $C_1$-$C_4$-Alkyl, Phenyl, Halogen, $R_7$ außerdem $C_5$-$C_{12}$-Alkyl, wenn $R_8$ H ist;
$R_7$ und $R_8$ gemeinsam auch ankondensierter $C_5$-$C_7$-Cycloalken- oder Benzolring;
$R_2$: H, $CH_3$, $C_2H_5$, i-$C_3H_7$, Cyclopropyl;
$R_3$: ($C_1$-$C_4$-Alkylen)-Het, wobei Het die Gruppen V, VI oder

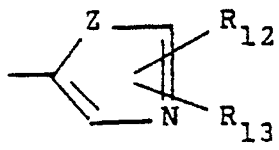 bedeutet,

worin

$R_{12}$: H, $CH_3$, Phenyl,

$R_{13}$: H, $CH_3$ und

Z: S, $NR_{12}$, CH=CH, N=CH oder CH=N ist;

$R_{14}$: H, $C_1$-$C_4$-Alkyl, $C_1$-$C_4$-Alkoxy, Halogen,

V: O, S.

Besonders hervorzuheben sind dabei diejenigen erfindungsgemäßen Verbindungen, in denen folgende Substituentenbedeutungen vorliegen:

$R_1$:

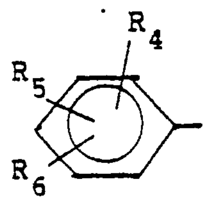

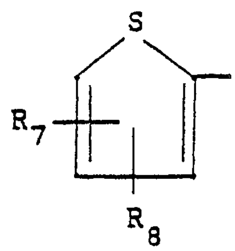

dabei stehen

$R_4$, $R_5$ für H, $CH_3$, $OCH_3$, Cl, F;

$R_4$ und $R_5$ gemeinsam auch für -O-$CH_2$-O-oder -O-$CH_2$-$CH_2$-O, gebunden an benachbarte C-Atome des Phenylrings ($R_6$ dann gleich H);

$R_6$ für H, $CH_3$, $OCH_3$;

$R_7$, $R_8$ für H, $C_1$-$C_4$-Alkyl, Cl, Br; gemeinsam auch für einen ankondensierten Cycloalkenring mit 5 oder 6 C-Atomen;

$R_2$: H, $CH_3$, $C_2H_5$, i-$C_3H_7$, Cyclopropyl;

$R_3$: $C_2$-$C_4$-Alkylen-Het, wenn Het für

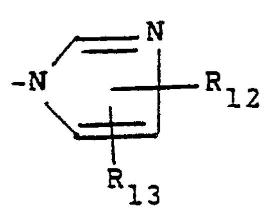 oder 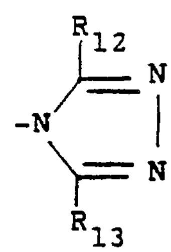

$R_{12}$: H, $CH_3$, $C_6H_5$

$R_{13}$: H, $CH_3$

und

$C_1$-$C_2$-Alkylen-Het, wenn Het für

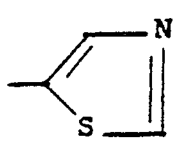 oder 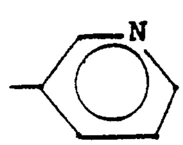 steht;

$R_{14}$: H, $C_1$-$C_4$-Alkyl;

V: O, S.

Die erfindungsgemäßen Verbindungen werden nach an sich bekannten Verfahren hergestellt. Dabei können die Methoden angewandt werden, die z.B. in "Chemistry of Heterocyclic compounds", Vol. 37, Wiley, New York (1981), S. 34 ff und 69 ff, beschrieben sind oder in F. Clemence et al. Eur. J. Med. Chem.-Chem. Ther. 20, 257-266 (1985).

1. 1-Acylamidrazone der Formel

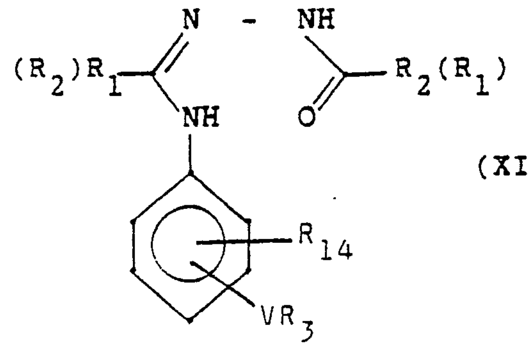

(XI),

in der $R_1$, $R_2$, $R_3$, $R_{14}$ und V die oben angegebene Bedeutung haben (die Substituenten $R_1$ und $R_2$ können, wie angedeutet, auch umgekehrt angeordnet sein), werden ohne Lösungsmittel oder in einem unter den Reaktionsbedingungen inerten Lösungsmittel erhitzt, wobei der Ringschluß zu I eintritt. Die Reaktionstemperatur beim Umsetzen in der Schmelze beträgt vorzugsweise 150-200 °C. Als Lösungsmittel werden zweckmäßig solche verwendet, deren Siedetemperatur in dem genannten Bereich liegt, so daß die Umsetzung unter Rückfluß stattfinden kann.

Die Verbindungen der Formel XI können nach üblichen Methoden erhalten werden, z.B. wie folgt:

(a) Iminoether werden mit Hydraziden zu substituierten Iminoethern umgesetzt, die mit Anilinen Acylamidrazone ergeben. Beim letzten Schritt können 1,3,4-Oxadiazole als Zwischenprodukte auftreten. Die weitere Umsetzung zu den Verbindungen der Formel I erfordert nicht unbedingt eine Zwischenisolierung der 1-Acylamidrazone.

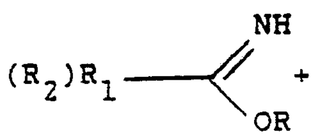

(XII)

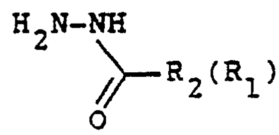

(XIII)

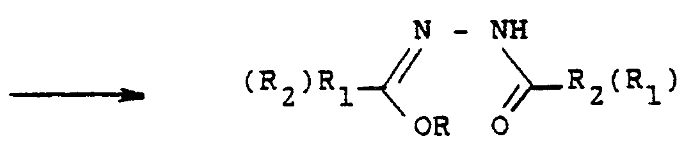

(XIV)

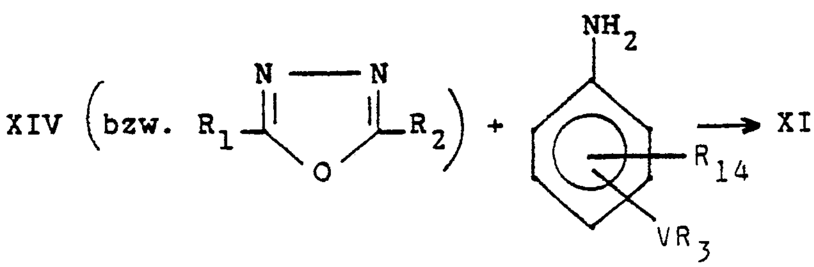


In den vorstehenden Formeln steht R für einen niederen Alkylrest, die anderen Symbole haben die oben

angegebenen Bedeutungen. $R_1$ und $R_2$ können, wie zuvor und im folgenden, jeweils umgekehrt angeordnet sein.

(b) Entsprechende Amidrazine werden mit Säurehalogeniden, Säureanhydriden bzw. Orthoestern umgesetzt:

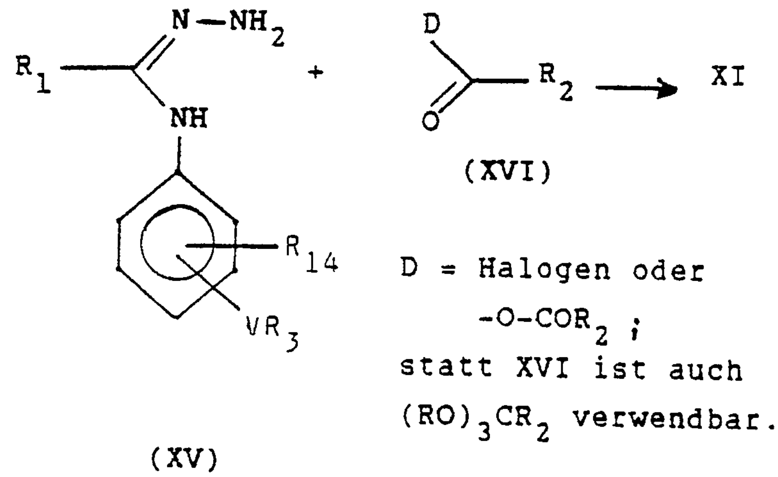


(XVI)

(XV)

(c) Thioamide bzw. S-Methylisothioamide werden mit Hydraziden umgesetzt:

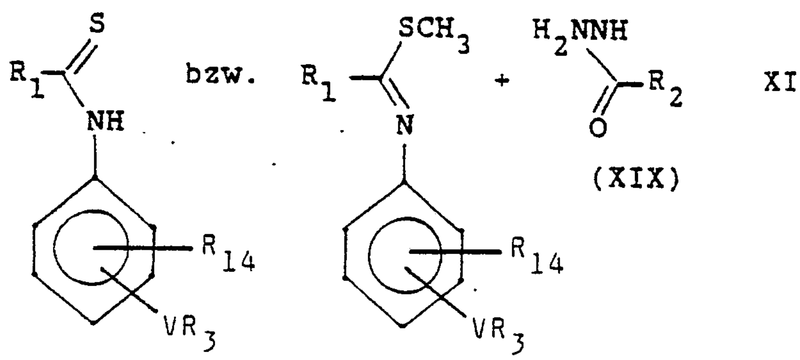

(XIX)

(XVII) (XVIII)

(d) Imidchloride werden mit Hydraziden umgesetzt:

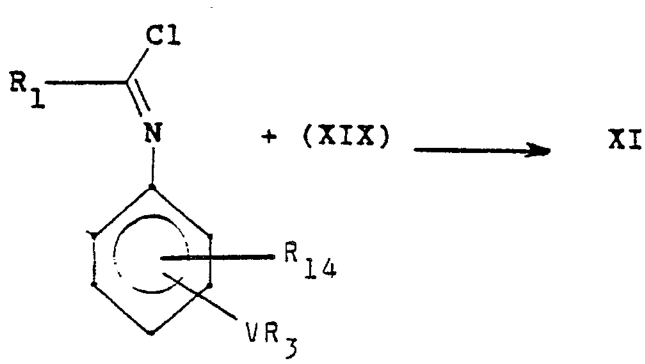

2. Verbindungen der Formel (I), in denen $R_2$ gleich $CH_3$ oder $C_2H_5$ ist, können durch Umsetzung von Amidrazonen mit Diethylamino- bzw. Ethoxyethinen oder -propinen bei Temperaturen zwischen 120-180 °C in aprotischen Lösungsmitteln wie z.B. Xylol erhalten werden:

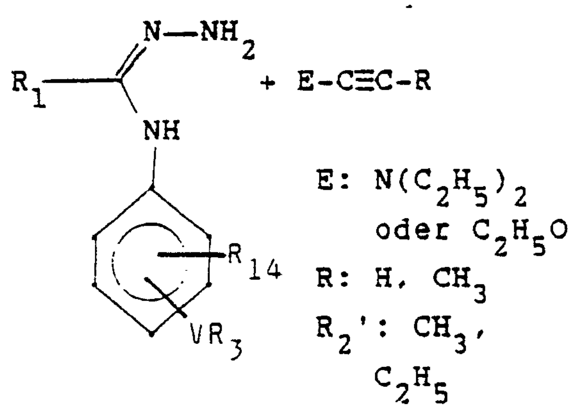


(XX)

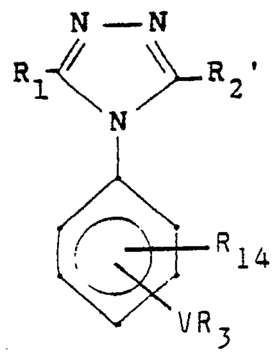

(XXI)

3. Verbindungen der Formel (I), in denen für $R_2$ ein niederes Alkyl steht, sind aus Hydrazonen (gebildet aus Amidrazonen mit β-Ketosäureestern oder β-Diketonen) durch Erhitzen im Schmelzzustand oder in aprotischen Lösungsmitteln wie Xylol zu erhalten:

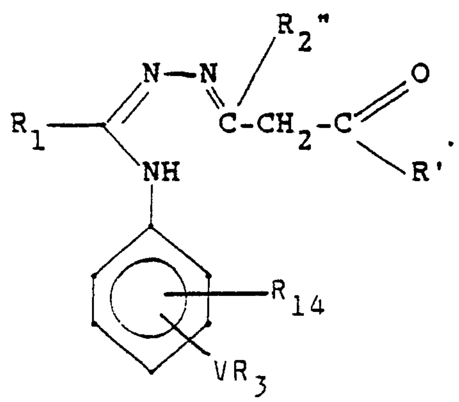

(XXII)

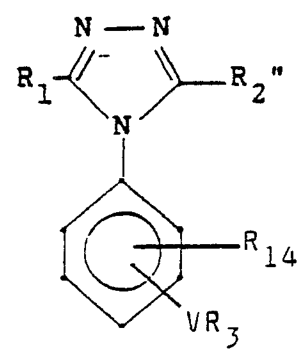

(XXIII)

R′: Alkyl oder Alkoxy (vorzugsweise mit 1 bis 8 C-Atomen)

$R''_2$: $C_1$-$C_4$-Alkyl

4. Verbindungen der Formel I, in denen in $R_3$ "Het" für eine der Gruppen V, VI, VII oder VIII steht, können durch Umsetzung von Verbindungen der Formel

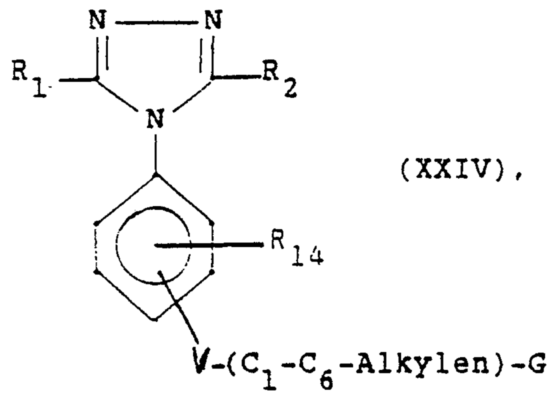

(XXIV),

in der $R_1$, $R_2$, $R_{14}$ und V die obige Bedeutung haben und G für eine "leaving group", vorzugsweise Halogen oder ein Sulfosäurerest, mit den V, VI, VII oder VIII entsprechenden Heterocyclen in polaren Lösungsmitteln umsetzt.
Der Heterocyclus wird vorzugsweise in Form eines Natriumsalzes eingesetzt. Als Lösungsmittel eignen sich z.B. Dimethylformamid (DMF), Dimethylsulfoxid (DMSO) oder Hexamethylphosphorsäuretriamid (HMPT). Die Reaktionstemperatur beträgt vorzugsweise 20-100° C.
Die Verbindungen der Formel XXIV können aus den Verbindungen der Formel

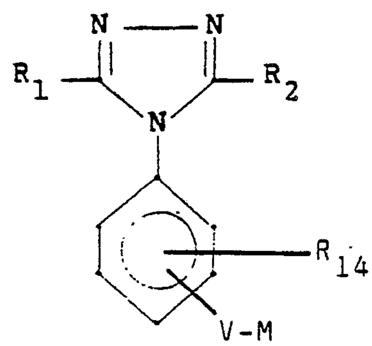

(XXV),

(M Alkalimetall, $R_1$, $R_2$ wie oben definiert) durch Umsetzung mit Verbindungen der Formel
Halogen-($C_1$-$C_4$-Alkylen)-G (XXVI)
erhalten werden.
Die Verbindungen der Formel XXV können aus den entsprechenden Methoxyverbindungen durch Entmethylierung und Überführung der erhaltenen Phenole in die Alkalisalze hergestellt werden. Die Phenole können nach bekannten Methoden in die Thiophenole umgewandelt und diese in die Alkalisalze überführt werden.

5. Umsetzung von Verbindungen der Formel XXV mit Verbindungen der Formel
G - $R_3$ (XXVII)
worin G und $R_3$ die obigen Bedeutungen haben. Die Umsetzung erfolgt in polaren Lösungsmitteln wie niedere Alkohole wie Ethanol, Ketone wie Methylethylketon, DMF, HMPT, DMSO, bei Raumtemperatur oder etwas erhöhter Temperatur.

6. Verbindungen der Formel I, in denen $R_3$ als Heterocyclus einen über die 4-Position verknüpften 4H-1,2,4-Triazolring enthält, können aus Aminen der Formel

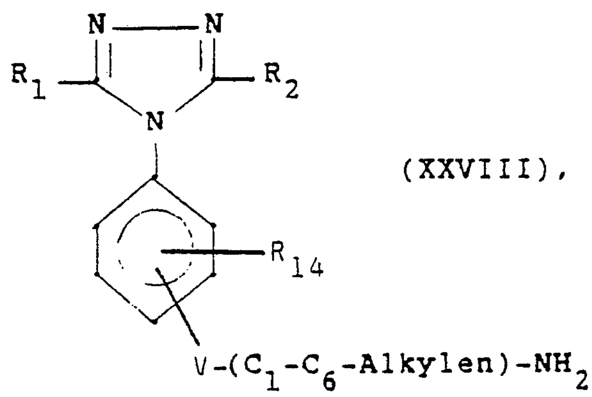

(XXVIII),

durch Ringschlußreaktion nach den in Verfahren 1, 2 und 3 angegebenen Methoden hergestellt werden.

Die unten beschriebenen Beispiele sollen die Verfahren näher erläutern.

Die erfindungsgemäßen Verbindungen sind wertvolle Arzneistoffe, insbesondere aufgrund PAF-antagonistischen Wirkung. Sie eignen sich daher vor allem zur Behandlung von Krankheiten, bei denen entzündliche und/oder allergische Vorgänge eine Rolle spielen, wie beispielsweise bei Asthma, aspirin-induziertem Asthma, chronischer Bronchitis, allergischer Rhinitis und ARDS (Adult Respiratory Distress Symptome); Entzündungen und Autoimmunkrankheiten wie rheumatoider Arthritis, Lupus erythematosus, multipler Sklerose, Glomerulonephritis, Psoriasis, Pruritus, Abstoßung von Nieren-, Leber- und anderen Transplantaten, entzündlichen Darmerkrankungen, Sklerodermie und anderen Autoimmunerkrankungen (etwa M. Werlhof); cardiovasculärer Erkrankungen wie Herzinfarkt, Reperfusionsschäden, Herzversagen, instabiler Angina pectoris, Schlaganfall, pulmonaler (mit Ischämie verbundene) Hypertonie, anderen ischämischen und hypoxischen Bedingungen; Schock und Schockprophylaxe, cardio-vasculären Störungen bei Schock (Sepsis, septischem Schock, polytraumatischem Schock, anaphylaktischem Schock); Organversagen bei Schock (ARDS, Nierenversagen, Gastrointestinalgeschwüren), DIC (Disseminated Intravascular Coagulation), Thrombose/Thromboembolie; ferner Ausbreitung von Metastasen, Agoraphobie, depressiven Erkrankungen, Nebenwirkungen von Arzneimitteln (immunologische Unverträglichkeit, ischämie-induzierender Wirkung, gewebeschädigender Wirkung). Sie können auch in Kombinationen eingesetzt werden, für die PAF-Antagonisten geeignet sind, etwa mit β-Adrenergika, Parasympatholytika, Corticosteroide, Antiallergika, Sekretolytika. Bei der Kombination mit TNF (Tumor-Nekrose-Faktor) wird eine bessere Verträglichkeit (Ausschaltung störender Nebenwirkungen) des TNF erreicht; TNF läßt sich daher gewünschtenfalls auch in höheren Dosen einsetzen als bei seiner alleinigen Anwendung. (Unter "Kombination" ist hier auch die Anwendung der beiden Wirkstoffe in getrennten Zubereitungen und in einem gewissen zeitlichen Abstand zu verstehen). Bei der gemeinsamen Anwendung der erfindungsgemäßen Verbindungen mit β-Adrenergika kann ein synergistischer Effekt erzielt werden, z.B. in der Broncholyse.

Als Maß für die PAF-antagonistische Wirkung seien die Hemmkonzentrationen ($IC_{50}$) bei der PAF-induzierten Thrombocytenaggregation angegeben (Verbindungen der Formel I mit $R_2$ gleich $CH_3$, $VR_3$ in para-Position zum Triazolylring und $R_{14}$ gleich H):

| $R_1$ | $-V-R_3$ | $IC_{50}$ x $10^{-6}$ |
|---|---|---|
| 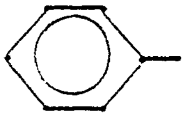 | 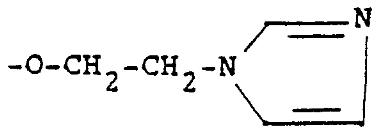 | 1,1 |
| 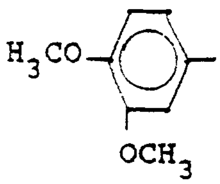 | 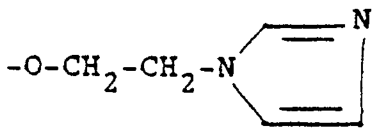 | 0,8 |
| 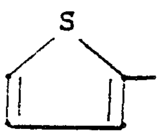 | 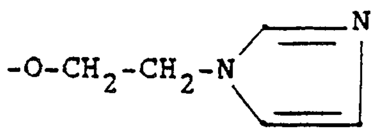 | 0,7 |
| 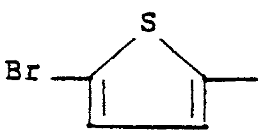 | 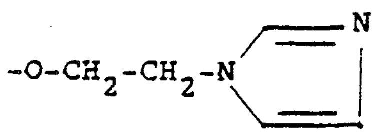 | 0,7 |
| 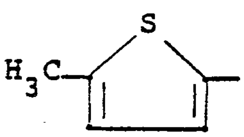 | 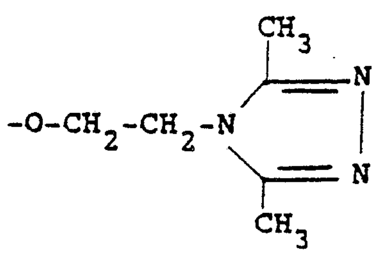 | 0,2 |
| 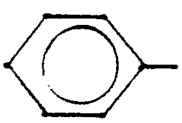 | 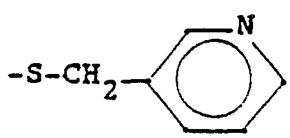 | 0,2 |
| 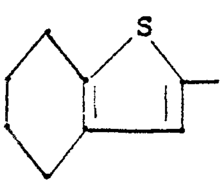 | 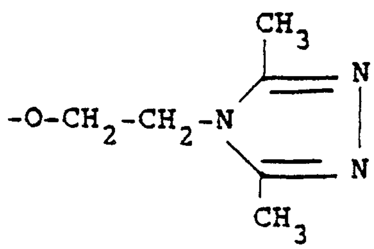 | 0,09 |

| $R_1$ | $-V-R_3$ | $IC_{50} \times 10^{-6}$ |
|---|---|---|
| 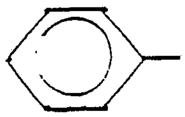 | 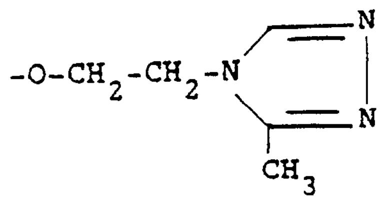 | 0,1 |

Die neuen Verbindungen können topisch, oral, transdermal, parenteral oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragées, Kapseln, Oblaten, Pulver, Lösungen, Suspensionen, Inhalationsaerosole, Salben, Emulsionen, Sirupe, Suppositorien.

Die therapeutische und prophylaktische Dosis ist abhängig von der Beschaffenheit und Ernsthaftigkeit des Krankheitszustandes.

Eine wirksame Dosis der erfindungsgemäßen Verbindungen liegt bei oraler Anwendung zwischen 1 und 100, vorzugsweise zwischen 10 und 80 mg/Dosis, bei intravenöser oder intramuskulärer Anwendung zwischen 0,001 und 50, vorzugsweise zwischen 0,1 und 30 mg/Dosis. Für die Inhalation sollen Lösungen, die 0,01 bis 1,0, vorzugsweise 0,1 bis 0,5 % Wirkstoff enthalten, eingesetzt werden.

Formulierungsbeispiele

1. Tabletten

| Zusammensetzung: | |
|---|---|
| Wirkstoff gemäß der Erfindung | 30 Gew.-Teile |
| Stearinsäure | 6 Gew.-Teile |
| Traubenzucker | 564 Gew.-Teile |

Die Bestandteile werden in üblicher Weise zu Tabletten von 600 mg Gewicht verarbeitet. Gewünschtenfalls kann der Wirkstoffgehalt erhöht oder vermindert und die Traubenzuckermenge entsprechend vermindert oder erhöht werden.

2. Suppositorien

| Zusammensetzung: | |
|---|---|
| Wirkstoff gemäß der Erfindung | 80 Gew.-Teile |
| Lactose, gepulvert | 45 Gew.-Teile |
| Kakao-Butter | 1575 Gew.-Teile |

Die Bestandteile werden in üblicher Weise zu Suppositorien von 1,7 g Gewicht verarbeitet.

3. Inhalationspulver

Mikronisiertes Wirkstoffpulver (Verbindung der Formel I; Teilchengröße ca. 0,5 bis 7 µm) werden in einer Menge von 0,02 mg mit 10 mg mikronisierter Lactose und gegebenenfalls geeigneten Mengen weiterer Wirkstoffe in Hartgelatinekapseln abgefüllt. Das Pulver wird aus üblichen Inhalationsgeräten, z.B. gemäß DE-A 3345 722, inhaliert.

4. Dosieraerosol

| | |
|---|---|
| Wirkstoff gemäß der Erfindung | 0,5 Gew.-% |
| Sorbitantrioleat | 0,5 Gew.-% |
| Monofluortrichlormethan und Difluordichlormethan (2:3) | 99,0 Gew.-% |

Die Mischung wird in Dosieraerosolgeräte üblicher Art abgefüllt. Die Dosiervorrichtung wird beispielsweise so ausgelegt, daß pro Hub 0,05 ml der Zubereitung abgegeben werden.

Beispiel 1

5-(5-Brom-2-thienyl)-4-[4-[2-[4-(3,5-dimethyl-1,2,4-triazolyl)]ethoxy]]phenyl-3-methyl-4H-1,2,4-triazol

3 g Essigsäure-[2-(5-brom-2-thenoyl)-hydrazonid]ethylester, Fp. 134-7°C (hergestellt aus 5-Bromthiophen-2-carbonsäurehydrazid, Fp. 142-4°C, durch Umsetzung mit Acetimidsäureethylester-hydrochlorid), und 2 g 4-[2-(4-Aminophenoxy)ethyl]-3,5-dimethyl-4H-1,2,4-triazol, Fp. 151-3°C (hergestellt durch Umsetzen von 2-(4-Nitrophenoxy)ethylamin mit Essigsäure-(2-acetylhydrazonid)ethylester und katalytische Reduktion der Nitroverbindung, Fp. 212-4°C, mit Pd/Kohle) werden unter Rühren 2 Stunden auf 150°C erwärmt. Der ölige Rückstand wird in 150 ml Chloroform/Methanol-Gemisch (4:1) gelöst, durch Zugabe von Aktivkohle und Kieselgel entfärbt und filtriert. Das Lösungsmittel wird abdestilliert, der Rückstand in 20 ml Essigester gelöst, abgekühlt, die ausgefallene Titelverbindung (2,1 g, 57 % d.Th.) wird abgesaugt und aus Acetonitril umkristallisiert; Fp. 224-6°C.

Beispiel 2

5-(3,4-Dimethoxyphenyl)-4-[4-[2-(1-imidazolyl)ethoxyphenyl-3-trifluormethyl-4H-1,2,4-triazol

3,4 g 3,4-Dimethoxybenzoesäure-(2-trifluoracetylhydrazonid)methylester (Fp. 103-4°C; hergestellt durch Umsetzen von 3,4-Dimethoxybenzoesäureiminomethylesterhydrochlorid und Trifluoressigsäurehydrazid) und 1-[2-(4-Aminophenoxy)ethyl]imidazol (Fp. 87-9°C; hergestellt durch Umsetzen von 2-(4-Nitrophenoxy)-p-toluolsulfonsäureethylester mit Imidazol und katalytische Reduktion der Nitroverbindung, Fp. Oxalat: 131-4°C, mit Pd/Kohle) werden 2 Stunden bei 150°C gerührt. Der ölige Rückstand wird in 50 ml Ethanol gelöst, über Aktivkohle filtriert und eingeengt. Der Rückstand wird durch Erwärmen in Essigester gelöst und mit Ether verdünnt. Die Titelverbindung fällt aus (1,5 g; 32,6 % d.Th.); sie wird abgesaugt und durch Umkristallisieren aus Essigester gereinigt, Fp. 165-7°C.

Beispiel 3

4-[4-[2-(1-Imidazolyl)ethoxy]]phenyl-3-methyl-5-(2-thienyl)-4H-1,2,4-triazol

1,95 g Imidazol werden in 25 ml Dimethylformamid gelöst, portionsweise mit 1,37 g Natriumhydriddispersion (55-60 %ig in Öl) versetzt und eine Stunde gerührt. Nach beendeter Wasserstoffentwicklung werden

8,3 g 4-[4-(2-Chlorethoxy)phenyl]-3-methyl-5-(2-thienyl)-4H-1,2,4-triazol (Fp. 149-51 ° C; hergestellt aus 4-(4-Hydroxyphenyl)-3-methyl-5-(2-thienyl)-4H-1,2,4-triazol, Fp. 278 ° C, mit p-Toluolsulfonsäure-(2-Chlorethylester) in Dimethylformamid zugegeben und unter Zusatz von Kaliumcarbonat 2 Stunden bei 80 bis 90 ° C gerührt. Das Dimethylformamid wird abdestilliert, der Rückstand in Chloroform gelöst, die Lösung mit verdünnter Natronlauge gewaschen, mit Natriumsulfat getrocknet und abdestilliert. Der Rückstand wird durch Erwärmen in Essigester gelöst, nach dem Abkühlen wird die ausgefallene Titelverbindung abgesaugt und aus Acetonitril umkristallisiert; Fp. 170-2 ° C. Das Hydrochlorid der Verbindung kristallisiert aus Isopropylalkohol/Ether bei Zugabe der berechneten Menge Salzsäure; Fp. 213-33 ° C (Zers.).

Beispiel 4

3-Methyl-4-[4-(3-pyridyl)methoxy]phenyl-5-(2-thieny)-4H-1,2,4-triazol

5,14 g 4-(4-Hydroxyphenyl)-3-methyl-5-(2-thienyl)-4H-1,2,4-triazol, 50 ml Ethanol, 40 ml 1N Natriumethylatlösung und 3,3 g 3-Picolylchlorid-hydrochlorid werden 5 Stunden unter Rückfluß erhitzt. Nach dem Abdestillieren des Ethanols wird der Rückstand in 150 ml Chloroform gelöst, die Lösung wird mit verdünnter Natronlauge gewaschen, getrocknet und abdestilliert. Der Rückstand wird in Essigester gelöst, die Lösung wird abgekühlt und die ausgefallene Titelverbindung abgesaugt. Durch Umkristallisieren aus Acetonitril erhält man die reine Verbindung, Fp. 158 ° C.

Beispiel 5

4-[4-[2-[4-(3,5-Dimethyl-1,2,4-triazolyl)]ethoxy]]phenyl-3-methyl-5-phenyl-4H-1,2,3,4-triazol

2,94 g 4-[4-(2-Aminoethoxy)phenyl-3-methyl-5-phenyl-4H-1,2,4-triazol, Fp. 125-6 ° C (hergestellt aus 4-(4-Hydroxyphenyl)-3-methyl-5-phenyl-4H-1,2,4-triazol durch Umsetzen mit N,N-Dibenzylaminoethylchlorid und katalytische Hyrierung mit Pd/Kohle) und 1,85 g Essigsäure-(2-acetylhydrazonid)-ethylester werden in 10 ml Ethanol 30 Minuten unter Rückfluß gekocht, anschließend wird das Ethanol abdestilliert und die Schmelze 30 Minuten auf ca. 150 ° C erwärmt. Der kristalline Rückstand wird in Ethanol gelöst, über Aktivkohle filtriert und das Lösungsmittel abdestilliert. Es bleibt ein Öl zurück, das in Acetonitril gelöst wird. Man kühlt ab und saugt die ausfallende Titelverbindung ab. Nach dem Umkristallisieren aus 95 %igem Acetonitril schmilzt die Verbindung (Hydrat) bei 245 ° C.

Beispiel 6

3-Methyl-4-[4-(3-pyridinylmethyl-thio)phenyl]-5-phenyl-4H-1,2,4-triazol

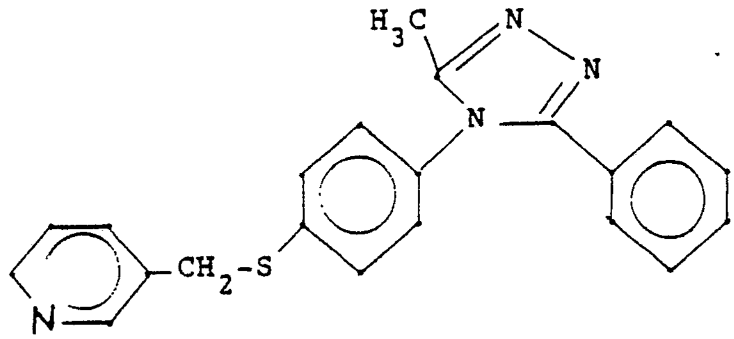

a) 3-[(4-Aminophenyl)thiomethyl]-pyridin

Eine Lösung von 25 g (0,2 mol) 4-Mercaptoanilin in 100 ml Ethanol wird unter Rühren zu einer Lösung von Natriumethylat in Ethanol [10,2 g (0,44 g-atom) Natrium in 200 ml absolutem Ethanol] getropft. Anschließend werden 32,8 g (0,2 mol) 3-Chlormethyl-pyridin-hydrochlorid portionsweise eingetragen, wobei die Temperatur auf 40-50 °C ansteigt. Die Reaktionsmischung wird über Nacht bei Raumtemperatur gerührt, mit Wasser versetzt und mit Methylenchlorid extrahiert. Die anorganische Phase wird über wasserfreiem Natriumsulfat getrocknet und am Rotationsverdampfer eingeengt. Das Rohprodukt wird in Methylenchlorid/Methanol 95:5 über eine Kieselgelsäule filtriert. Die nach dem Abdestillieren der Lösungsmittel zurückbleibenden Kristalle werden aus Acetonitril/Diisopropyläther umkristallisiert.
Ausbeute: 31 g (72 % d.Th.); Fp. 120-123 °C.

b) 3-[(4-Benzoylaminophenyl)thiomethyl]pyridin

Zu 17,3 g (0,08 mol) [(4-Aminophenyl)thiomethyl]pyridin und 8,91 g (0,088 mol) Triäthylamin in 250 ml Chloroform tropft man bei 20-25 °C eine Lösung von 11,25 g (0,08 mol) Benzoylchlorid in 50 ml Chloroform. Nach beendeter Zugabe wird die Reaktionsmischung zwei Stunden bei Raumtemperatur gerührt, mit 200 ml Wasser und anschließend mit 300 ml Petroläther (40-60 °C) versetzt. Die anfallenden Kristalle werden abgetrennt, in Essigester suspendiert, abgesaugt und getrocknet.
Ausbeute: 20,4 g (79 % d.Th.);
Fp. 148-150 °C.

c) 3-Methyl-4-[4-(3-pyridinylmethyl-thio)-phenyl]-5-phenyl-4H-1,2,4-triazol

Eine Suspension von 9,6 g (0,03 mol) des nach b) erhaltenen Produktes und 6,25 g (0,03 mol) Phosphorpentachlorid in 150 ml Toluol wird vier Stunden bei Rückflußtemperatur gerührt. Anschließend wird die Reaktionsmischung eingedampft, 100 ml Toluol hinzugefügt und wieder zur Trockne eingeengt. Der Rückstand wird in 200 ml Toluol suspendiert. Nach der Zugabe von 5.56 g (0,075 mol) Acethydrazid und 5.56 g (0,075 mol) Triäthylamin wird die Reaktionsmischung fünf Stunden am Wasserabscheider zum Rückfluß erhitzt und bei vermindertem Druck eingedampft. Der Rückstand wird zwischen Methylenchlorid und Wasser verteilt. Die organische Phase wird abgetrennt, nacheinander mit 5%iger Essigsäure, verdünntem Ammoniak und Wasser gewaschen und über Natriumsulfat getrocknet. Das nach dem Abdestillieren des Lösungsmittels zurückbleibende Öl wird an Kieselgel mit Aceton als Eluens chromatographiert. Man erhält 3,9 g (36% d.Th.) der obengenannten Verbindung;
Fp. 118-119 °C (Acetonitril/Diisopropyläther).

Analog den vorstehenden Beispielen lassen sich die in den nachstehenden Tabellen aufgeführten Verbindungen herstellen:

## Tabelle I

Verbindungen der Formel

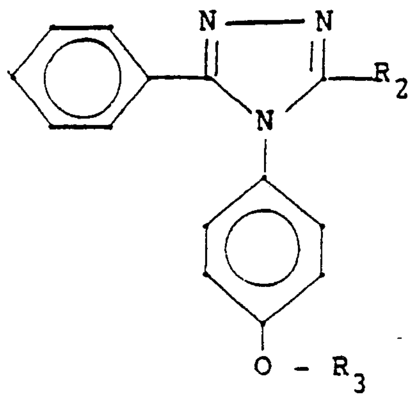

| Nr. | $R_2$ | $R_3$ | Fp.[°C] für Base | Fp.[°C] für Salz mit |
|---|---|---|---|---|
| 1 | H | $-(CH_2)_2-$ 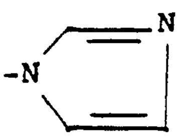 | 127-9 | |
| 2 | $CH_3$ | $-(CH_2)_2-$ 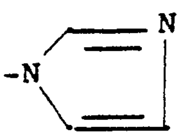 | 105 | 190-2 (HCl) |
| 3 | $CH_3$ | $-(CH_2)_3$ 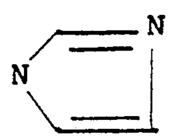 | 118-21 | 206-9 (HCl) |

| Nr. | $R_2$ | $R_3$ | Fp.[°C] für Base | Fp.[°C] für Salz mit |
|---|---|---|---|---|
| 4 | H | 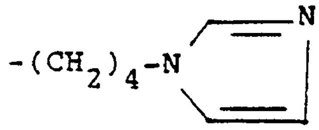 | 116 | |
| 5 | $CH_3$ | 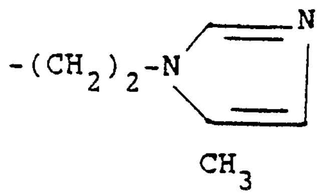 | 140-2 | 125-30 (HCl + $H_2O$) |
| 6 | $CH_3$ | 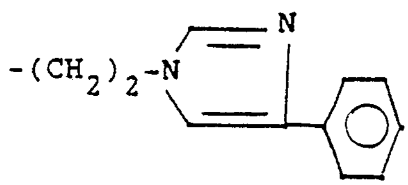 | 184-6 | 180-4 (2 HCl) |
| 7 | $CH_3$ | 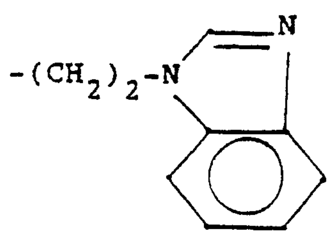 | 175-6 | 262-4 (HCl) |
| 8 | $CH_3$ | 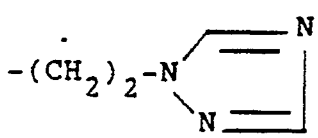 | 126-8 | |
| 9 | $CH_3$ | 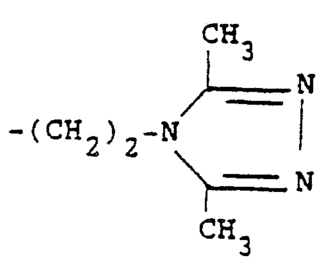 | 245 (Hydrat) | |

| Nr. | $R_2$ | $R_3$ | Fp.[°C] für Base | Salz mit |
|---|---|---|---|---|
| 10 | $CH_3$ | 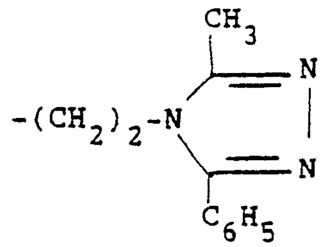 | 168-70 | |
| 11 | $CH_3$ | 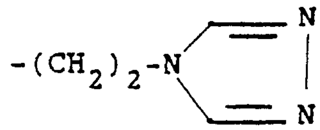 | 92-5 (Hydrat) | |
| 12 | $CH_3$ | 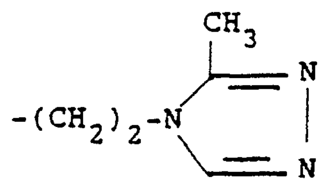 | 178-80 | |
| 13 | H | 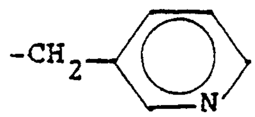 | 120-2 | |
| 14 | $CH_3$ | 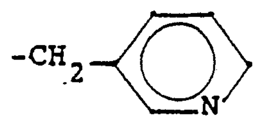 | 162-3 | |
| 15 | $C_2H_5$ | 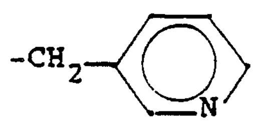 | 118-9 | |
| 16 | $i-C_3H_7$ | 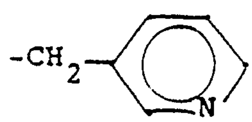 | 145-6 | |

| Nr. | $R_2$ | $R_3$ | Fp.[°C] für | |
|---|---|---|---|---|
| | | | Base | Salz mit |
| 17 | 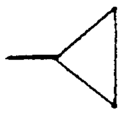 | $-CH_2-$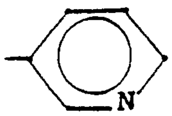 | 93-6 (Hydrat) | |
| 18 | 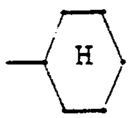 | $-CH_2-$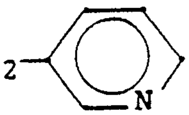 | 139-41 | |
| 19 | $CH_3$ | $-CH_2-$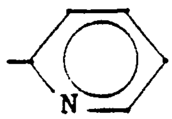 | 126-7 | |
| 20 | $CH_3$ | $-CH_2-$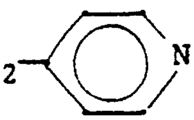 | 138-9 | |
| 20 | $CH_3$ | $-(CH_2)_2-$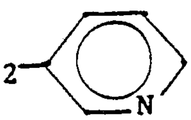 | 91-2 | |
| 21 | $C_2H_5$ | $-(CH_2)_2-$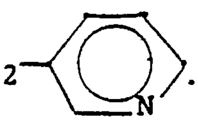 | | |
| 22 | $CH_3$ | $-(CH_2)_2-$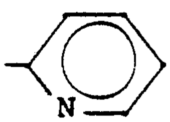 | 127-9 | |
| 23 | $i-CH_3H_7$ | $-(CH_2)_2-$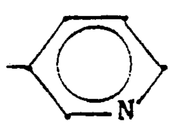 | 124-5 | |

| Nr. | $R_2$ | $R_3$ | Fp.[°C] für Base | Salz mit |
|---|---|---|---|---|
| 24 | $CF_3$ | $-CH_2-$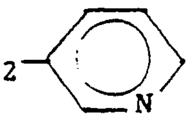 | 141-2 | |

## Tabelle II

Verbindungen der Formel

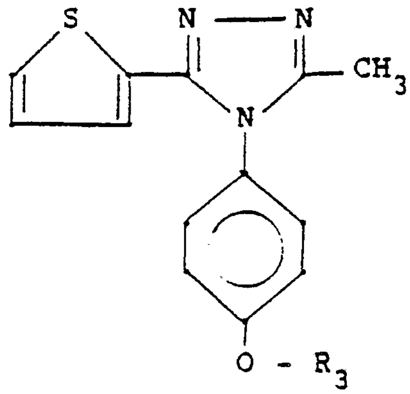

| Nr. | $R_3$ | Fp.[°C] für Base | Salz mit |
|---|---|---|---|
| 1 | 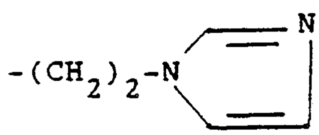 | 170-2 | 231-3 (HCl) |
| 2 | 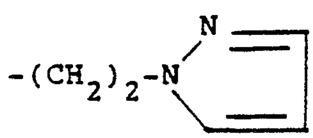 | 145-7 | |
| 3 | 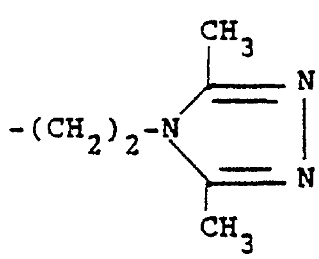 | 175-8 (Hydrat) | |
| 4 | 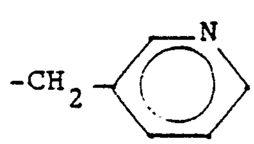 | 158 | |

| Nr | $R_3$ | Fp.[°C] für Base | Fp.[°C] für Salz mit |
|---|---|---|---|
| 5 | 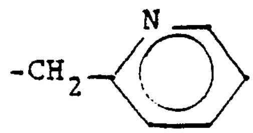 | 126-8 | |
| 6 | 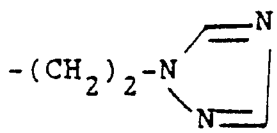 | 145-7 | |
| 7 | 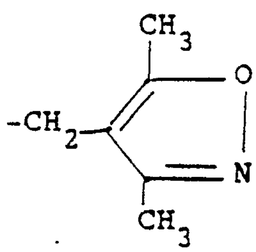 | 207-9 | |
| 8 | 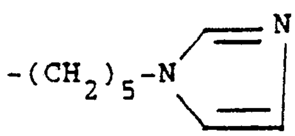 | | 192-4 (HCl) |
| 9 | 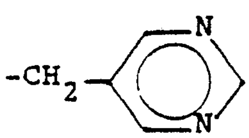 | | |
| 10 | 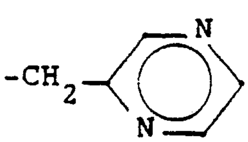 | | |
| 11 | 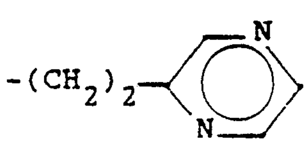 | | |

## Tabelle III

Verbindungen der Formel

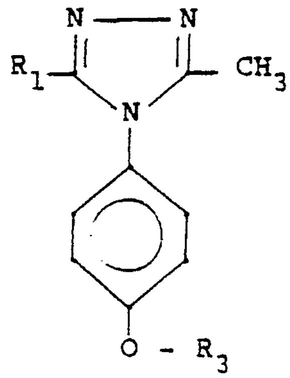

| Nr. | $R_1$ | $R_3$ | Fp.[°C] für Base | Salz mit |
|---|---|---|---|---|
| 1 | 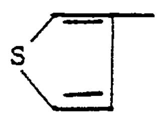 | $-(CH_2)_2-$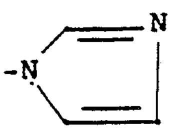 | 134-6 | |
| 2 | 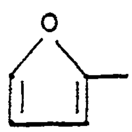 | $-(CH_2)_2-$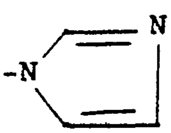 | 158-60 | |
| 3 | 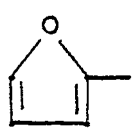 | $-CH_2-$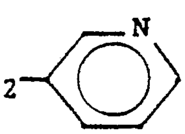 | 190-1 | |
| 4 | 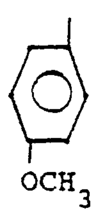 | $-(CH_2)_2-$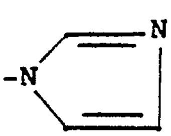 | 95 (Hydrat) | |

| Nr. | $R_1$ | $R_3$ | Fp.[°C] für Base | Salz mit |
|---|---|---|---|---|
| 5 | 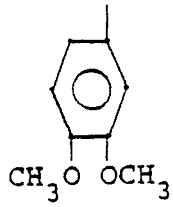 | 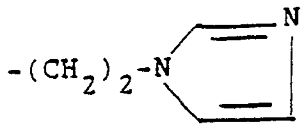 | 130 | |
| 6 | 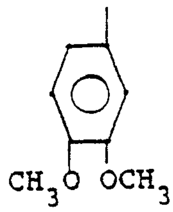 | 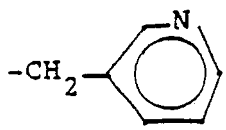 | 202-4 (2 HCl x $H_2O$) | |
| 7 | 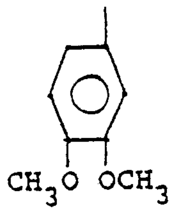 | 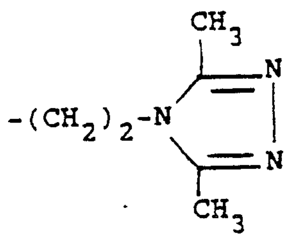 | 194-6 (x 1/2 $H_2O$) | |
| 8 | 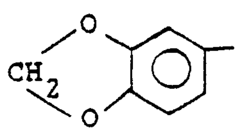 | 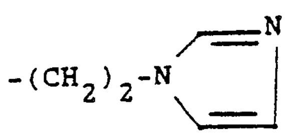 | 134-5 | |
| 9 | 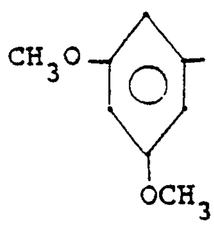 | 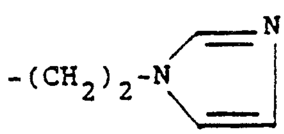 | 145-6 | |
| 10 | 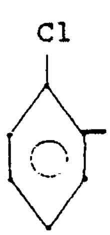 | 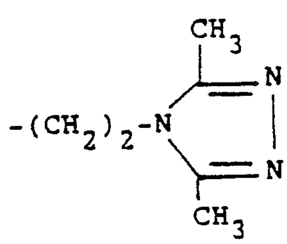 | 154-6 (x2$H_2O$) | |

| Nr. | $R_1$ | $R_3$ | Fp.[°C] für Base | Salz mit |
|---|---|---|---|---|
| 11 | 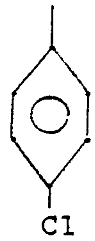 | 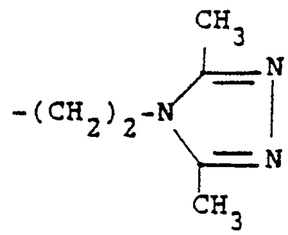 | 235 (x 1/2 $CH_3OH$) | |
| 12 | 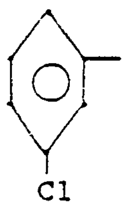 | 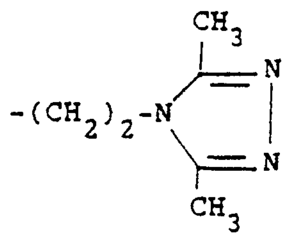 | 184-5 | |
| 13 | 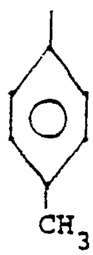 | 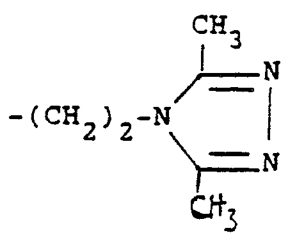 | 218-20 | |
| 14 | 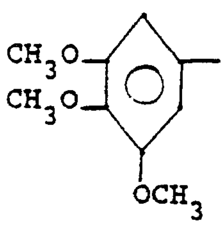 | 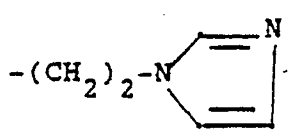 | 121-3 (x $H_2O$) | |
| 15 | 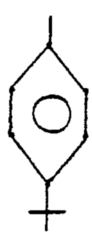 | 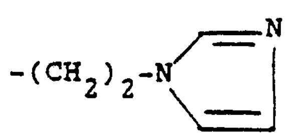 | 155-7 | |
| 16 | 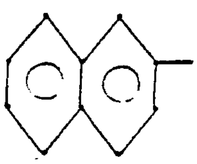 | 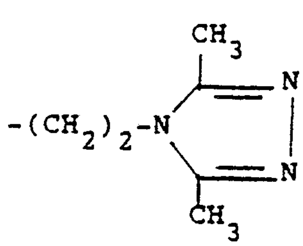 | 195-7 | |

| Nr. | $R_1$ | $R_3$ | Fp.[°C] für Base | Salz mit |
|---|---|---|---|---|
| 17 | 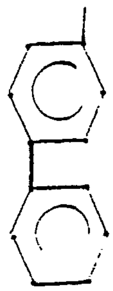 | 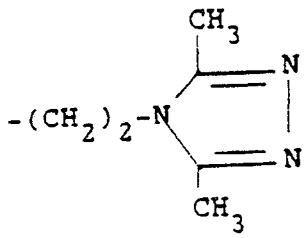 | 258-60 (x $CH_3OH$) | |
| 18 | 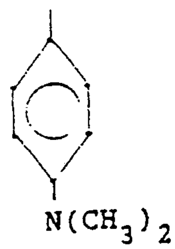 | 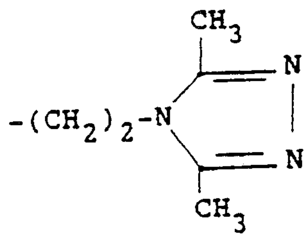 | 213-5 (x $H_2O$) | |
| 19 | 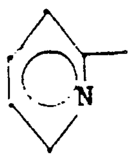 | 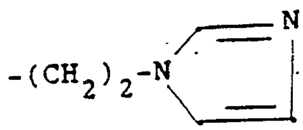 | 112-4 | |
| 20 | 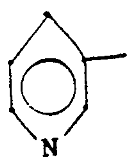 | 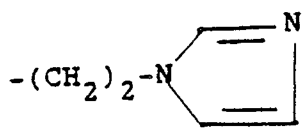 | | 192-4 (HCl) |
| 21 | 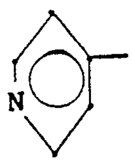 | 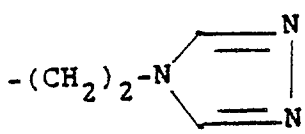 | 160-2 | |
| 22 | 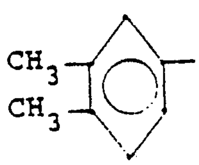 | 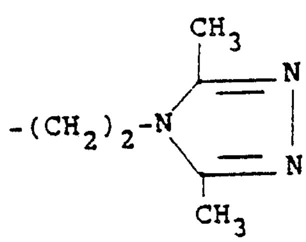 | 230-3 | |

| Nr. | $R_1$ | $R_3$ | Fp.[°C] für | |
|---|---|---|---|---|
| | | | Base | Salz mit |
| 23 | 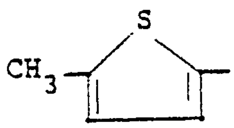 | 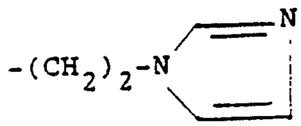 | 130-8 | |
| 24 | 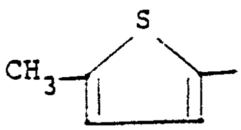 | 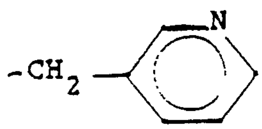 | 168-70 | |
| 25 | 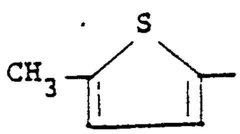 | 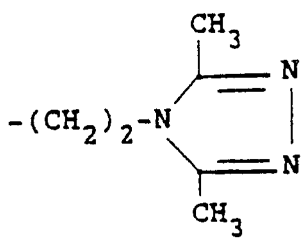 | 212-4 | |
| 26 | 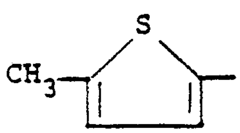 | 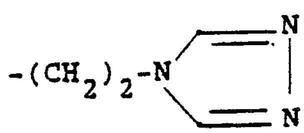 | 134-6 | |
| 27 | 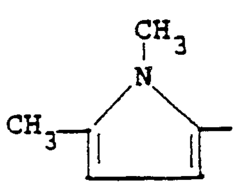 | 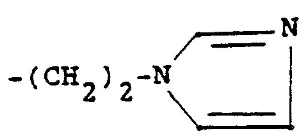 | 122-4 | |
| 28 | 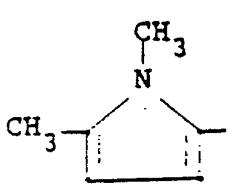 | 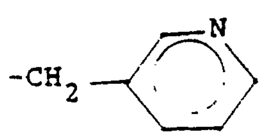 | 129-31 | |

| Nr. | $R_1$ | $R_3$ | Fp.[°C] für Base | Salz mit |
|---|---|---|---|---|
| 29 | 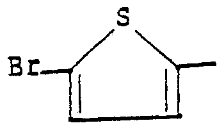 | 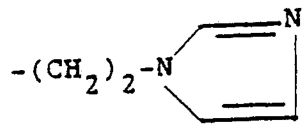 | 118-20 | |
| 30 | 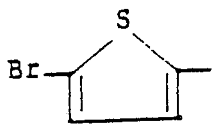 | 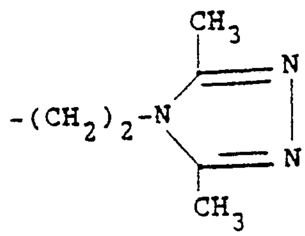 | 224-6 | |
| 31 | 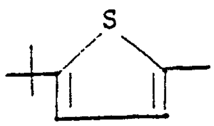 | 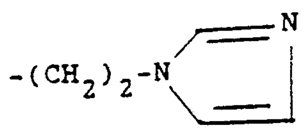 | 118-20 | |
| 32 | 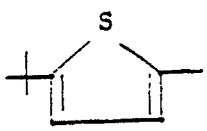 | 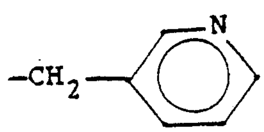 | 100-2 | |
| 33 | 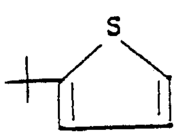 | 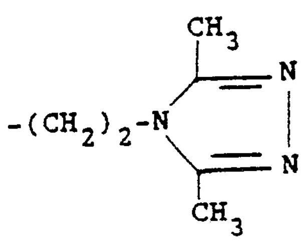 | 151-3 (x 1 Essigester) | |
| 34 | 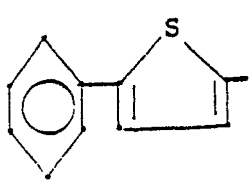 | 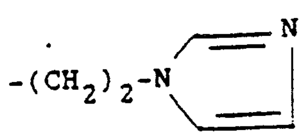 | 155-7 | |
| 35 | 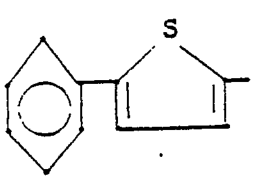 | 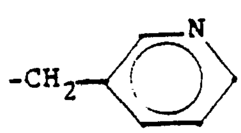 | 163-5 | |

| Nr. | $R_1$ | $R_3$ | Fp.[°C] für Base | Salz mit |
|---|---|---|---|---|
| 36 | 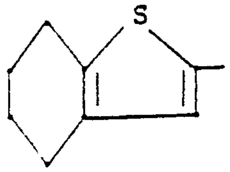 | $-CH_2-$ 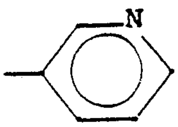 | 167-8 | |
| 37 | 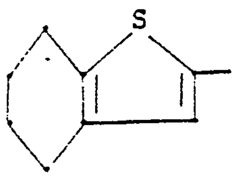 | $-(CH_2)_2-$ 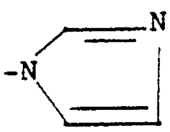 | 169-70 | |
| 38 | 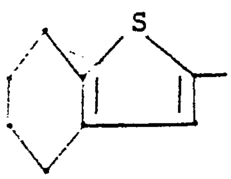 | $-(CH_2)_2-$ 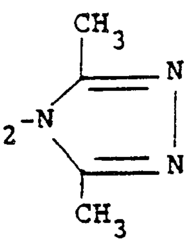 | 232-4 | |
| 39 | 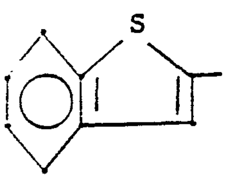 | $-(CH_2)_2-$ 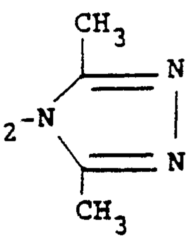 | 243-5 | |
| 40 | 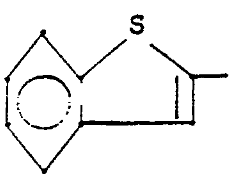 | $-(CH_2)_2-$ 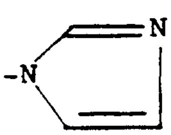 | 157-9 | |
| 41 | 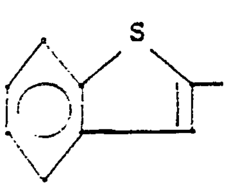 | $-(CH_2)_2-$ 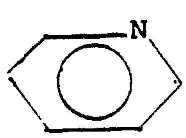 | 181-3 | |

| Nr. | $R_1$ | $R_3$ | Fp.[°C] für Base | Salz mit |
|---|---|---|---|---|
| 42 | 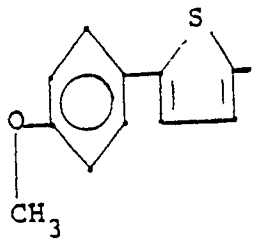 | $-CH_2-$ 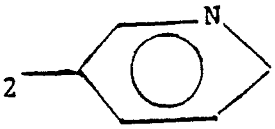 | 95-7 | |
| 43 | 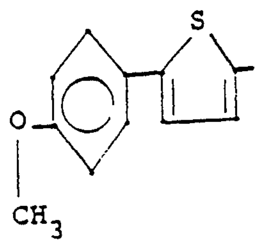 | $-(CH_2)_2-$ 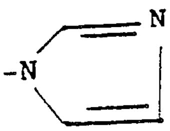 | 145-7 | |
| 44 | 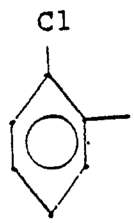 | $-CH_2-$ 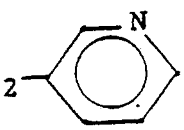 | 116-7 (Hemi-hydrat) | |
| 45 | 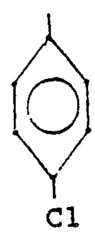 | $-CH_2-$ 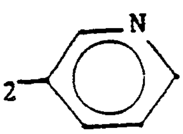 | 156-8 | |
| 46 | 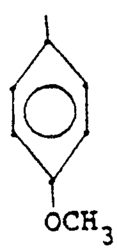 | $-CH_2-$ 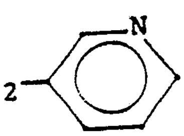 | 141-2 | |

| Nr. | $R_1$ | $R_3$ | Fp.[°C] für Base | Salz mit |
|---|---|---|---|---|
| 47 | 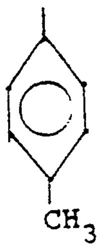 | -$CH_2$- 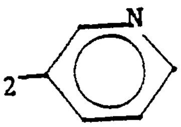 | 178-80 | |
| 48 | 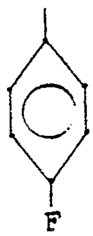 | -$CH_2$- 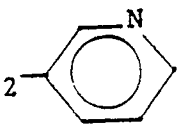 | 140-2 | |
| 49 | 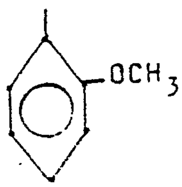 | -$CH_2$- 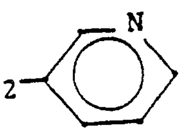 | 78-80 (Hemi-hydrat) | |
| 50 | 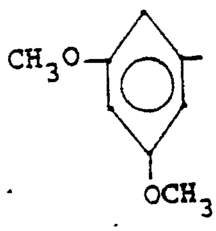 | -$CH_2$ 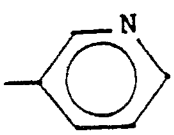 | 146-48 | |
| 51 | 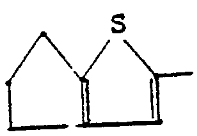 | -$(CH_2)_2$- 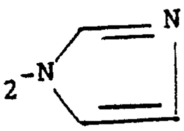 | 130-2 | |
| 52 | 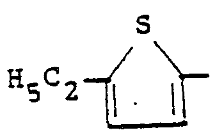 | -$(CH_2)_2$- 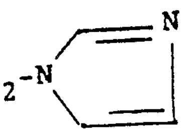 | 140-2 | |

| Nr. | $R_1$ | $R_3$ | Fp.[°C] für Base | Salz mit |
|---|---|---|---|---|
| 53 | 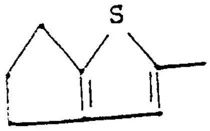 | 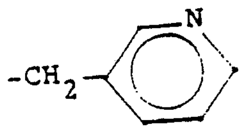 | 155-6 | |
| 54 | 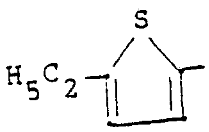 | 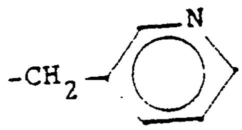 | 105 | |
| 55 | 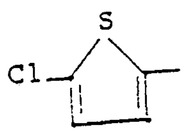 | 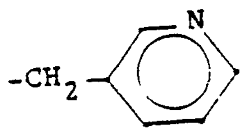 | 160-2 | |
| 56 | 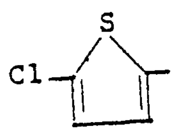 | 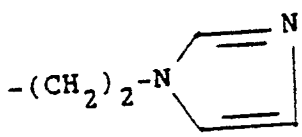 | 123-5 | |
| 57 | 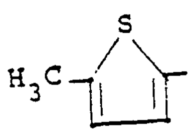 | 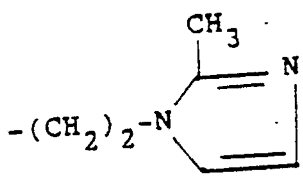 | | |
| 58 | 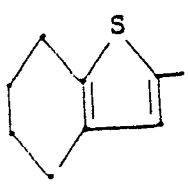 | 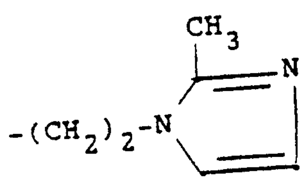 | | |
| 59 | 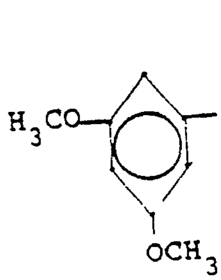 | 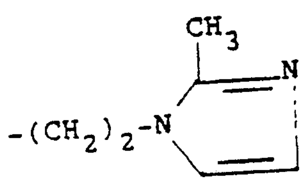 | | |

| Nr. | $R_1$ | $R_3$ | Fp.[°C] für Base | Fp.[°C] für Salz mit |
|---|---|---|---|---|
| 60 | 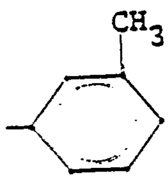 | 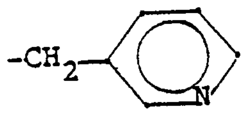 | 162-3 | |
| 61 | 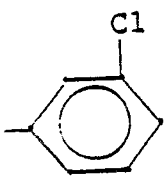 | 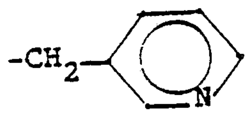 | 175-6 | |
| 62 | $-C_6H_5$ | 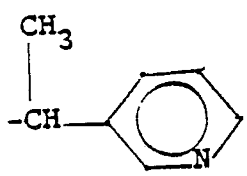 | 112-4 (Zers.) | |
| 63 | 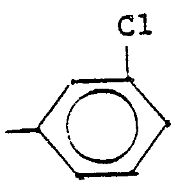 | 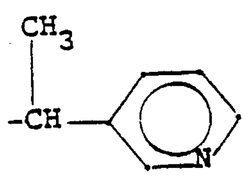 | | 132 (Zers.) (Fumarsäure) |
| 64 | 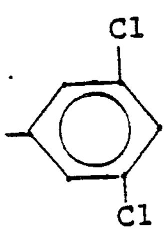 | 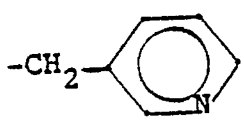 | | |
| 65 | 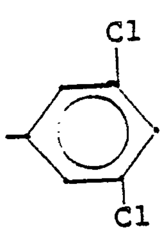 | 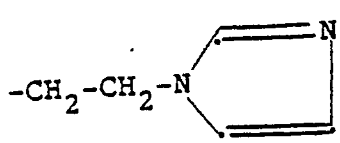 | | |

| Nr. | $R_1$ | $R_3$ | Fp.[°C] für Base | Salz mit |
|---|---|---|---|---|
| 66 | 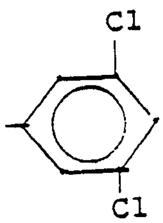 | 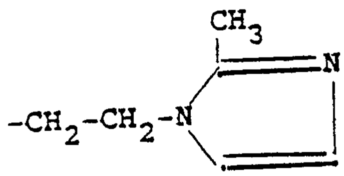 | | |
| 67 | 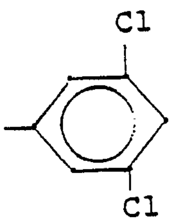 | 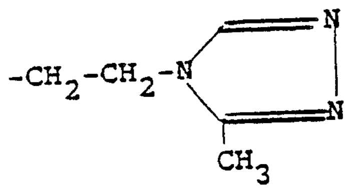 | | |
| 68 | 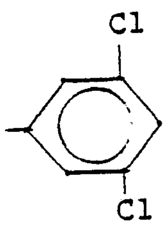 | 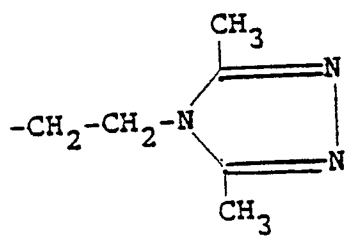 | | |
| 69 | 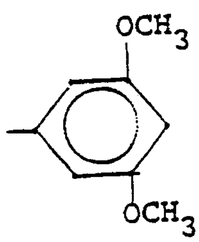 | 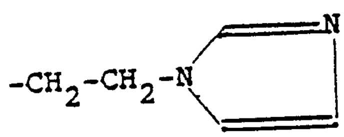 | | |
| 70 | 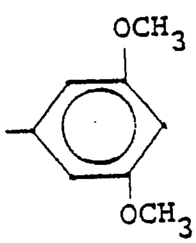 | 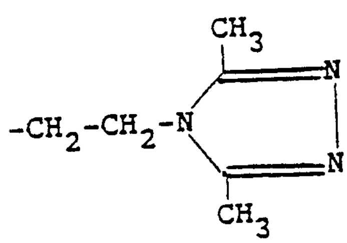 | | |

| Nr. | $R_1$ | $R_3$ | Fp. [°C] für Base | Salz mit |
|---|---|---|---|---|
| 71 | 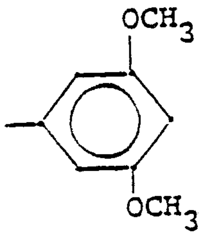 | 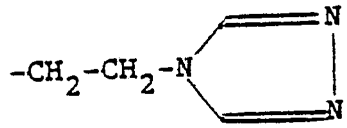 | 141-3 | |
| 72 | 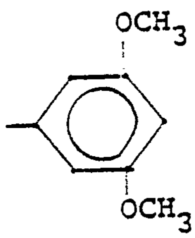 | 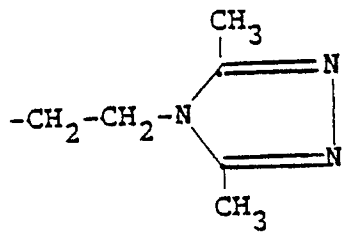 | 166-9 | |
| 73 | 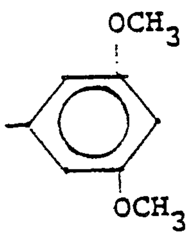 | 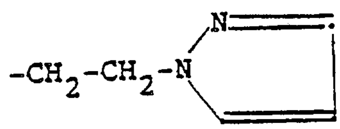 | 116-8 | |
| 74 | 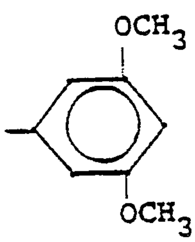 | 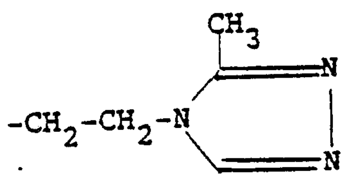 | 145-7 | |
| 75 | $C_6H_5$ | 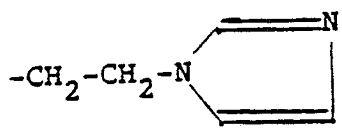 | | |

| Nr. | $R_1$ | $R_3$ | Fp. [°C] für Base | Fp. [°C] für Salz mit |
|---|---|---|---|---|
| 76 | 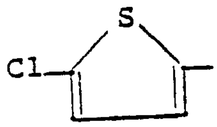 | 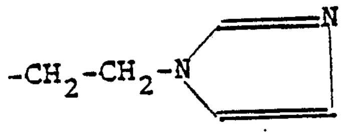 | 209-11 | |
| 77 | 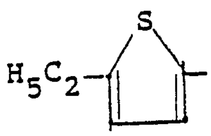 | 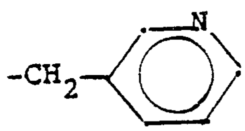 | 105 | |
| 78 | 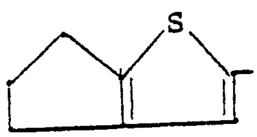 | 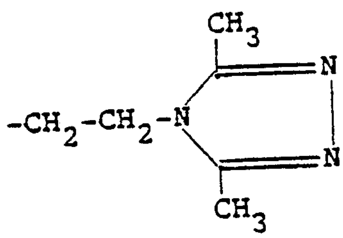 | 225-8 | |
| 79 | 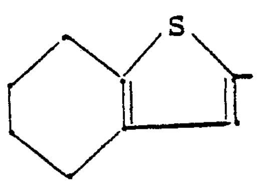 | 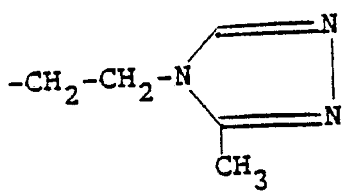 | 191-3 | |
| 80 | 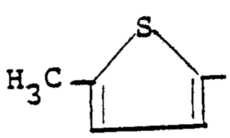 | 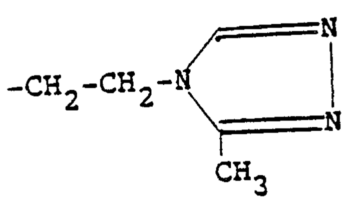 | 191-2 | |
| 81 | 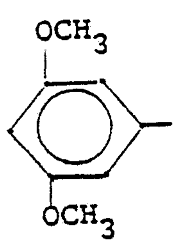 | 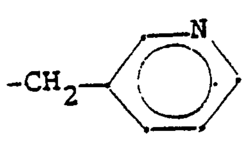 | 146-8 | |

| Nr. | $R_1$ | $R_3$ | Fp.[°C] für | |
|---|---|---|---|---|
| | | | Base | Salz mit |
| 82 | 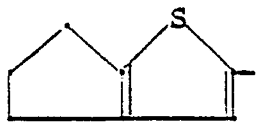 | 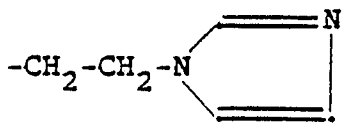 | 130-2 | |
| 83 | 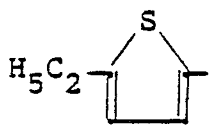 | 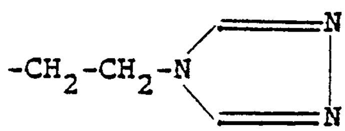 | 140-2 | |
| 84 | 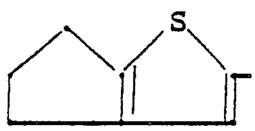 | 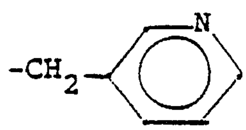 | 155-6 | |

TABELLE IV

Verbindungen der Formel

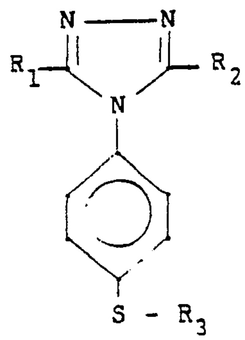

| Nr. | $R_2$ | $R_1$ | $R_3$ | Fp. [°C] Base | Salz mit |
|---|---|---|---|---|---|
| 1 | $CH_3$ | 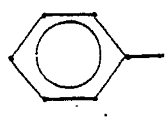 | $-CH_2-$ 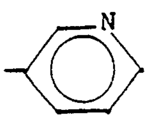 | 118-9 | |
| 2 | $i-C_3H_7$ | 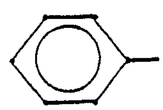 | $-CH_2-$ 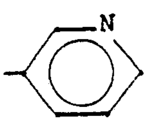 | 113-5 | |
| 3 | $CH_3$ | 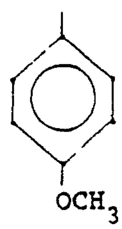 | $-CH_2-$ 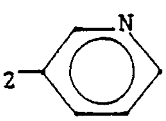 | 151-3 | |
| 4 | $CH_3$ | 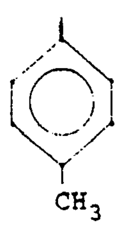 | $-CH_2-$ 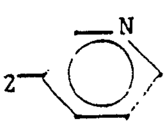 | 167-8 | |

| Nr. | $R_2$ | $R_1$ | $R_3$ | Fp. [°C] Base | Salz mit |
|---|---|---|---|---|---|
| 5 | $CH_3$ | 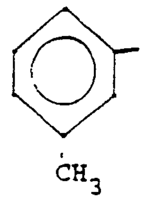 | 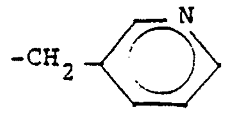 | 118-9 | |
| 6 | $CH_3$ | 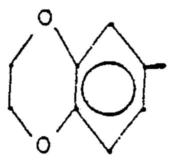 | 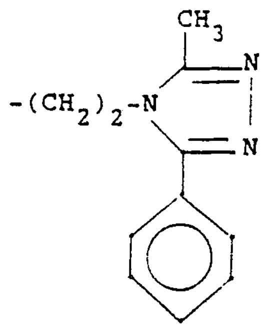 | | |
| 7 | $CH_3$ | 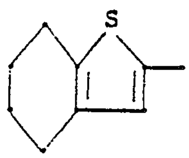 | 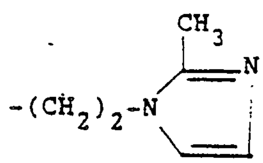 | | |
| 8 | $CH_3$ | 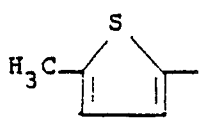 | 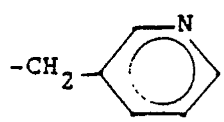 | | |
| 9 | $CH_3$ | 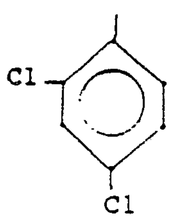 | 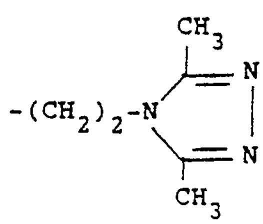 | | |

| Nr. | $R_2$ | $R_1$ | $R_3$ | Fp. [°C] Base | Salz mit |
|---|---|---|---|---|---|
| 10 | $CH_3$ | 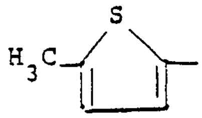 | 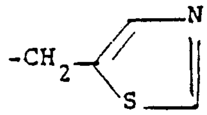 | | |
| 11 | $C_2H_5$ | 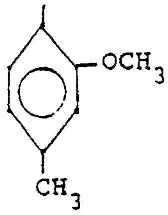 | 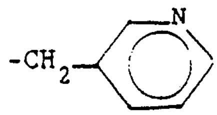 | | |
| 12 | $CF_3$ | 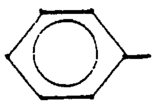 | 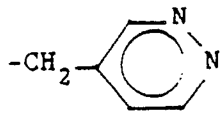 | | |
| 13 | 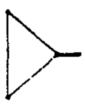 | 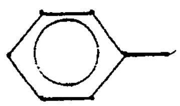 | 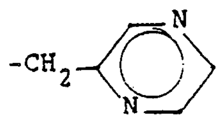 | | |
| 14 | $CH_3$ | 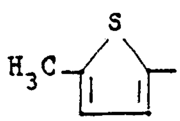 | 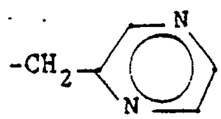 | | |
| 15 | $CH_3$ | 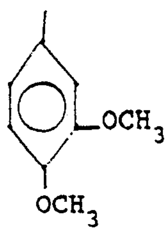 | 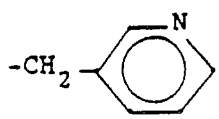 | | |
| 16 | $CH_3$ | 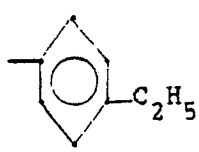 | 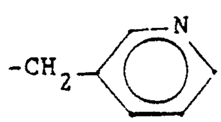 | | |

| Nr. | $R_2$ | $R_1$ | $R_3$ | Fp. [°C] Base | Salz mit |
|---|---|---|---|---|---|
| 17 | $CH_3$ | 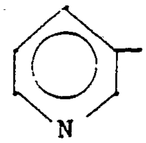 | 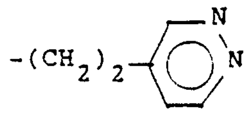 | | |
| 18 | $CH_3$ | 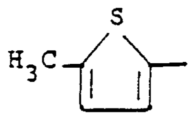 | 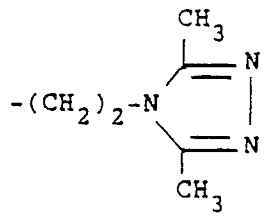 | | |
| 19 | $CH_3$ | 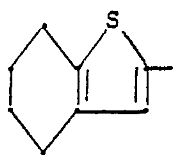 | 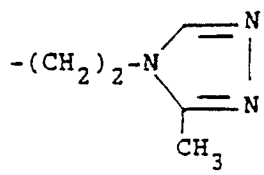 | | |
| 20 | $CH_3$ | 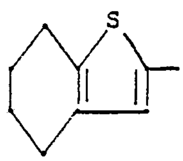 | 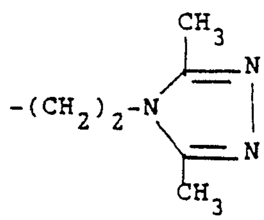 | | |
| 21 | $i-C_3H_7$ | 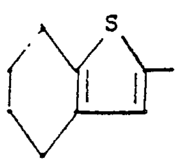 | 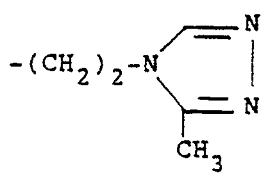 | | |
| 22 | $i-C_3H_7$ | 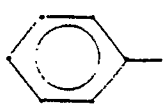 | 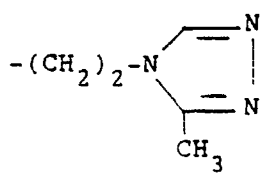 | | |

| Nr. | $R_2$ | $R_1$ | $R_3$ | Fp. [°C] Base | Salz mit |
|---|---|---|---|---|---|
| 23 | 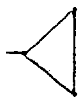 | 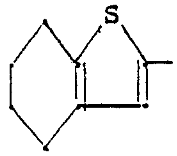 | $-(CH_2)_2-$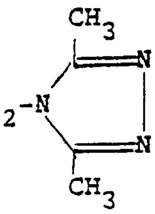 | | |
| 24 | $CH_3$ | 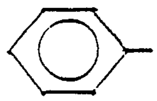 | $-(CH_2)_2-$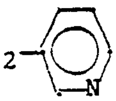 | | 155-6 (1,5 Fumarsäure) |
| 25 | i-$C_3H_7$ | 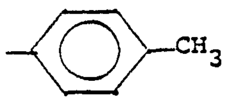 | $-(CH_2)_2-$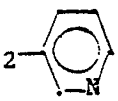 | | |
| 26 | $CH_3$ | 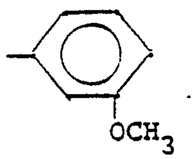 | $-CH_2-$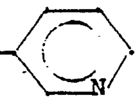 | 100-1 | |
| 27 | 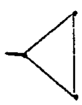 | 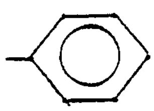 | $-CH_2-$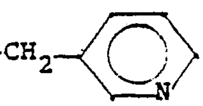 | | |
| 28 | i-$C_3H_7$ | 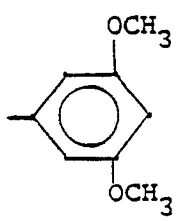 | $-CH_2-$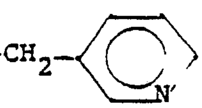 | | |
| 29 | i-$C_3H_7$ | 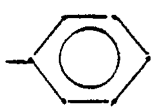 | $-(CH_2)_2-$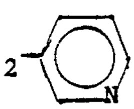 | | |

| Nr. | $R_2$ | $R_1$ | $R_3$ | Fp. [°C] Base | Salz mit |
|---|---|---|---|---|---|
| 30 | $CH_3$ | 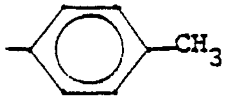 | $-(CH_2)_2-$ 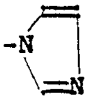 | | |
| 31 | $i-C_3H_7$ | 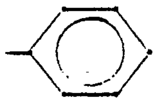 | $-(CH_2)_2-$ 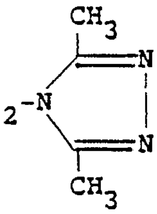 | | |
| 32 | $CH_3$ | 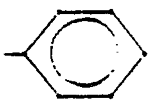 | $-(CH_2)_2-$ 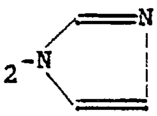 | | |
| 33 | $i-C_3H_7$ | 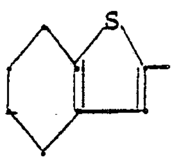 | $-(CH_2)_2-$ 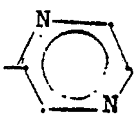 | | |
| 34 | $CH_3$ | 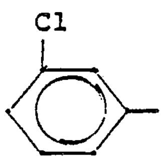 | $-CH_2$ 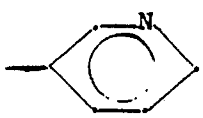 | 147-9 | |
| 35 | $CH_3$ | 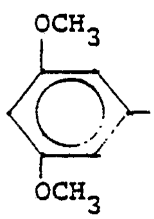 | $-CH_2$ 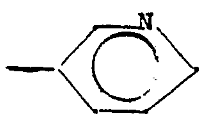 | | |

| Nr. | $R_2$ | $R_1$ | $R_3$ | Fp. [°C] Base | Salz mit |
|---|---|---|---|---|---|
| 36 | $CH_3$ | 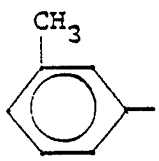 | 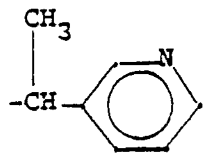 | 98-100 (Zers.) | |
| 37 | $CH_3$ | 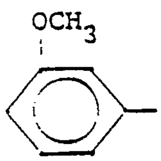 | 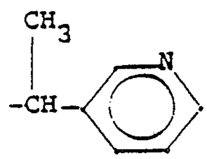 | | 126 (Zers.) (Fumarsäure) |
| 38 | $CH_3$ | $C_6H_5$ | 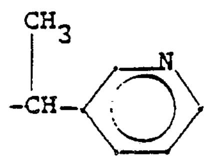 | | 146-8 (Zers.) (Fumarsäure) |

Erfindungsgemäß können ferner erhalten werden

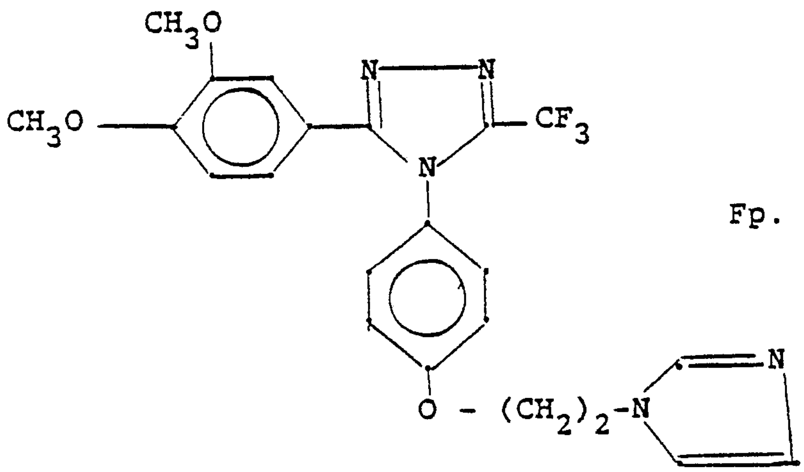

Fp. 165-7°C

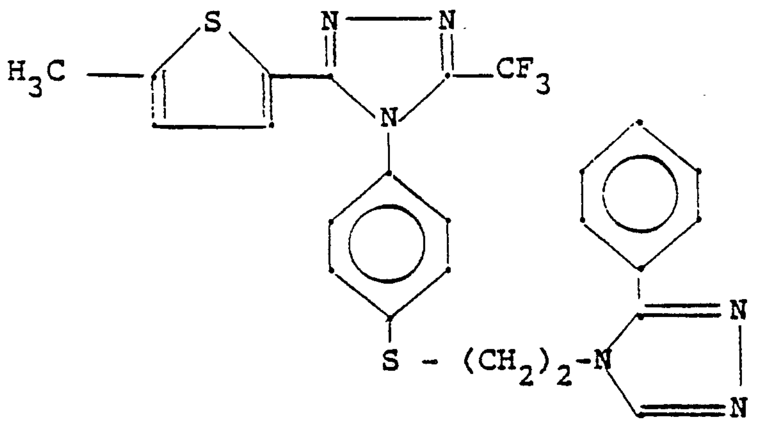

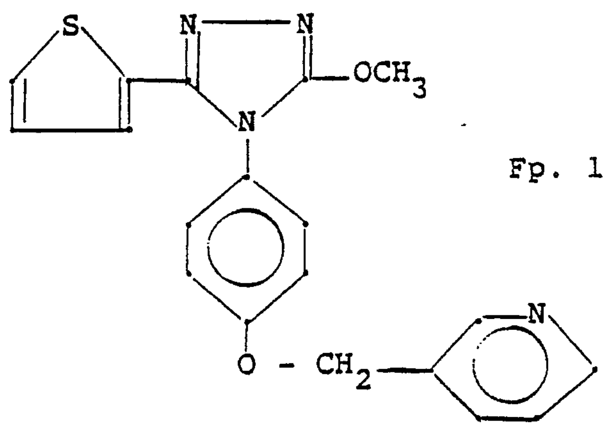

Fp. 144-6°C

Weitere Verbindungen der Formel I, die erfindungsgemäß erhalten werden können:

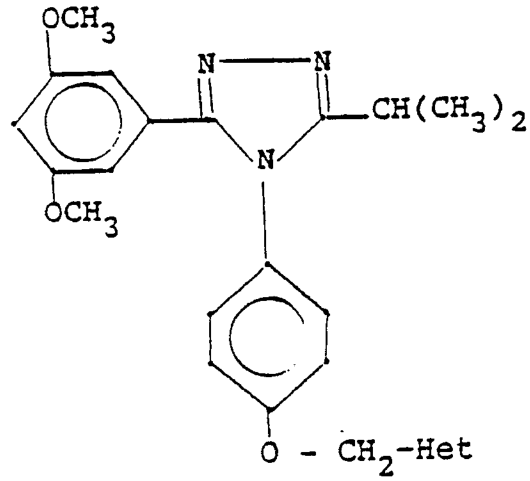

Het: 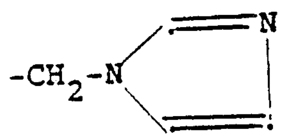

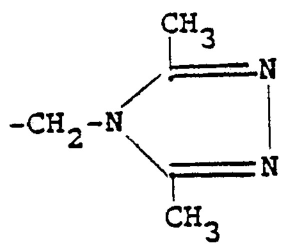

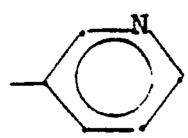

**Ansprüche**

1. Verbindungen der Formel

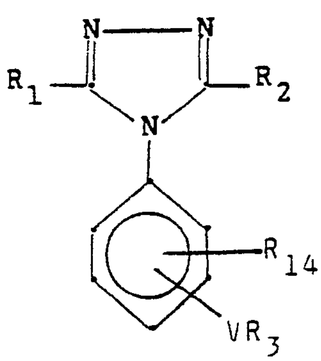

(I),

worin $R_1$, $R_2$, $R_3$, $R_{14}$ und V folgende Bedeutungen haben:

$R_1$:

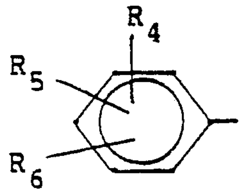

,

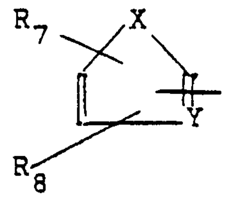

(II) (III)

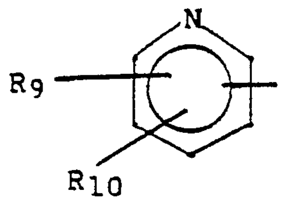

(IV),

wobei

$R_4$, $R_5$ für H, $R_{11}$, $OR_{11}$, Halogen, $CF_3$, Aryl, $N(R_{11})_2$, gemeinsam auch für einen ankondensierten $C_5$-$C_7$-Cycloalken-, einen ankondensierten Benzolring oder eine der an benachbarte C-Atome des Benzolrings gebundene Gruppen -N=CH-NH-, -N=CH-CH=CH-, -O-$CH_2$-O-, -O-$CH_2$-$CH_2$-O-, -NH-CO-NH- und -N=CH-CH=N-,

$R_6$ für H, $R_{11}$ oder $OR_{11}$ oder Aryl;

$R_7$ für H, $R_{11}$, Halogen oder Aryl;

$R_8$ für H, $R_{11}$ oder Halogen, $R_7$ und $R_8$ gemeinsam auch für einen ankondensierten $C_5$-$C_7$-Cycloalken- oder Benzolring,

$R_9$, $R_{10}$ für H, $R_{11}$, $OR_{11}$, $N(R_{11})_2$, gemeinsam auch für einen ankondensierten Benzolring;

X für S, O, NH oder N($C_1$-$C_4$-Alkyl);

Y für CH, N;

$R_{11}$ für $C_1$-$C_4$-Alkyl, und, wenn neben $R_{11}$ bzw. einer $R_{11}$ enthaltenden Gruppe die Reste II, III bzw. IV keine weiteren Substituenten aufweisen, auch $C_5$-$C_{12}$-Alkyl oder -Alkoxy stehen;

$R_2$: H, $C_1$-$C_4$-Alkyl, $CF_3$, $C_1$-$C_4$-Alkoxy, $CH_2$-O-($C_1$-$C_4$-Alkyl), $C_3$-$C_6$-Cycloalkyl; $R_3$ ($C_1$-$C_6$-Alkylen)-Het, wobei Het

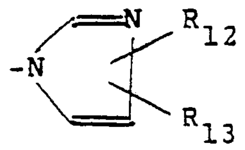, 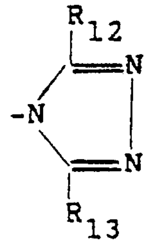, 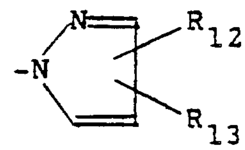

(V) (VI) (VII)

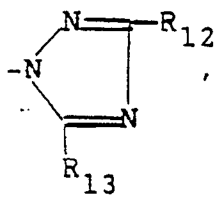, 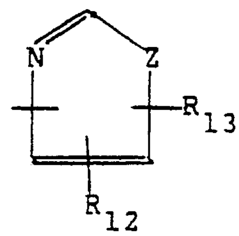, 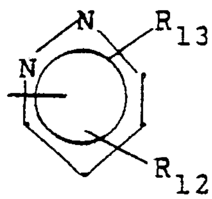

(VIII) (IX) (X)

Z: S, N-$R_{12}$, CH=CH, N=CH bzw. CH=N;
$R_{12}$: H, $C_1$-$C_4$-Alkyl oder Aryl;
$R_{13}$: H, $C_1$-$C_4$-Alkyl, $R_{12}$ und $R_{13}$ gemeinsam gegebenenfalls einen ankondensierten Benzolring bedeuten;
$R_{14}$: H, $C_1$-$C_4$-Alkyl, $C_1$-$C_4$-Alkoxy oder Halogen bedeutet, und auch zweimal vorhanden sein kann;
V: O oder S;
sowie die Salze dieser Verbindungen mit Säuren.

2. Verbindungen nach Anspruch 1, worin
$R_1$ für die Gruppen II und III,
wobei
$R_4$, $R_5$ und $R_6$ H, $C_1$-$C_4$-Alkyl, $C_1$-$C_4$-Alkoxy, Halogen, $R_4$ auch $C_5$-$C_{12}$-Alkyl oder -Alkoxy, wenn $R_5$ und $R_6$ H sind,
$R_4$ und $R_5$ gemeinsam auch ankondensiertes $C_5$-$C_7$-Cycloalken, Methylendioxy, Ethylendioxy sowie, falls $R_6$ H ist, auch einen ankondensierten Benzolring bedeuten;
X S, O, NH, N($C_1$-$C_4$-Alkyl) bedeutet;
Y N, wenn X NH oder N-($C_1$-$C_4$-Alkyl) ist,
CH, wenn X S oder O bedeutet;
$R_7$, $R_8$ H, $C_1$-$C_4$-Alkyl, Phenyl, Halogen,
$R_7$ außerdem $C_5$-$C_{12}$-Alkyl, wenn $R_8$ H ist;
$R_7$ und $R_8$ gemeinsam auch ankondensierter $C_5$-$C_7$-Cycloalken- oder Benzolring bedeuten;
$R_2$ für H, $CH_3$, $C_2H_5$, i-$C_3H_7$, Cyclopropyl;
$R_3$ für ($C_1$-$C_4$-Alkylen)-Het, wobei Het die Gruppen V, VI oder

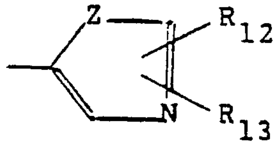

bedeutet,

worin
$R_{12}$: H, $CH_3$, Phenyl,
$R_{13}$: H, $CH_3$ und
Z: S, $NR_{12}$, CH=CH, N=CH oder CH=N ist,

$R_{14}$ für H, $C_1$-$C_4$-Alkyl, $C_1$-$C_4$-Alkoxy oder Halogen,
V für O, S steht.

3. Verbindungen nach Anspruch 1, in denen
$R_1$:

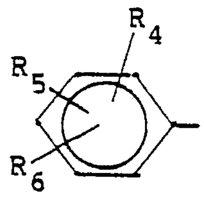 oder 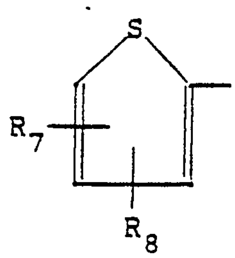 ist,

wobei
$R_4$, $R_5$ für H, $CH_3$, $OCH_3$, Cl, F;
$R_4$ und $R_5$ gemeinsam auch für -O-$CH_2$-O- oder -O-$CH_2$-$CH_2$-O, gebunden an benachbarte C-Atome des Phenylrings ($R_6$ dann gleich H);
$R_6$ für H, $CH_3$, $OCH_3$;
$R_7$, $R_8$ für H, $C_1$-$C_4$-Alkyl, Cl, Br; gemeinsam auch für einen ankondensierten Cycloalkenring mit 5 oder 6 C-Atomen stehen;
$R_2$: H, $CH_3$, $C_2H_5$, i-$C_3H_7$, Cyclopropyl ist;
$R_3$: $C_2$-$C_4$-Alkylen-Het ist, wenn Het für

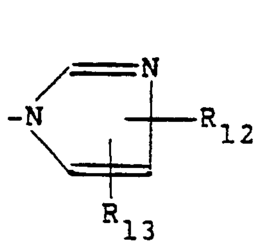 oder 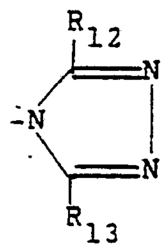 ,

$R_{12}$ für H, $CH_3$, $C_6H_5$,
$R_{13}$ für H, $CH_3$ steht;
und
$C_1$-$C_2$-Alkylen-Het ist, wenn Het für

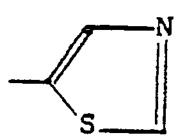 oder 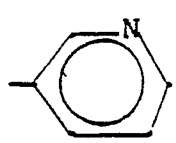 steht;

$R_{14}$ für H steht;
V O, S ist.

4. Verbindungen nach Anspruch 1, 2 oder 3, in denen
$R_1$ für

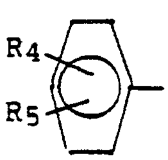 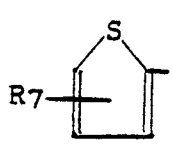 , 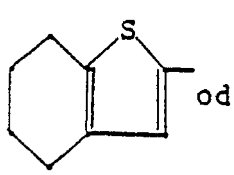 oder 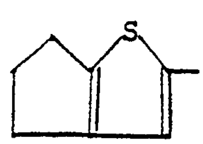

($R_4$: H, $CH_3$, $OCH_3$, Br, Cl, F,

$R_5$: H, $CH_3$, $OCH_3$, Cl,
$R_7$: H, $CH_3$, Cl, Br);
$R_2$ für $CH_3$, $C_2H_5$, i-$C_3H_7$ oder Cyclopropyl;
$R_3$ für

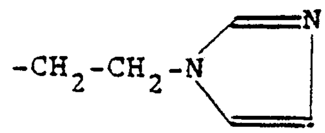,

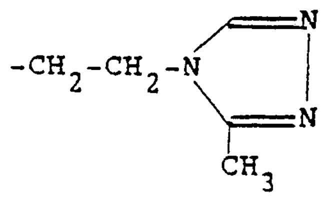,

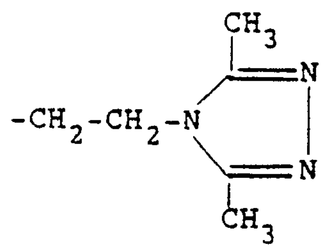,

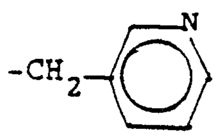 oder 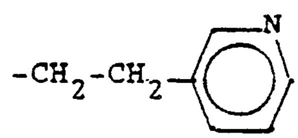

$R_{14}$ H ist
und
V für O oder S steht.

5. Pharmazeutische Zubereitungen, gekennzeichnet durch einen Gehalt an einer Verbindung nach Anspruch 1, 2, 3 oder 4 neben üblichen Hilfs- und/oder Trägerstoffen.

6. Verwendung von Verbindungen nach Anspruch 1, 2, 3 oder 4 zur Behandlung von Krankheiten, insbesondere von entzündlichen und allergischen Vorgängen, an denen PAF beteiligt ist.

7. Verfahren zur Herstellung von Verbindungen nach Anspruch 1, 2, 3 oder 4 nach an sich bekannten Verfahren, dadurch gekennzeichnet, daß man
a) durch Erhitzen von 1-Acylamidrazonen der Formel

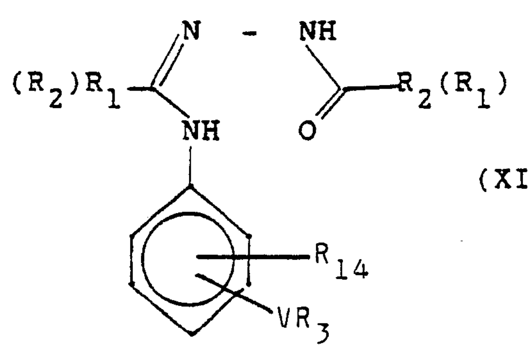

(XI),

in der $R_1$, $R_2$, $R_3$, $R_{14}$ und V die oben angegebene Bedeutung haben, erhitzt oder daß man

b) ein Amidrazon der Formel

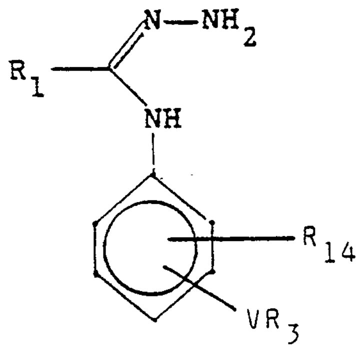

(XX),

in der $R_1$, $R_3$, $R_{14}$ und V die obige Bedeutung haben, mit einem Ethinderivat der Formel

E-C≡C-R

in der E $N(C_2H_5)_2$ oder $OC_2H_5$, R H oder $CH_3$ bedeutet, unter Ringschluß umsetzt

oder daß man

c) ein Hydrazon der Formel

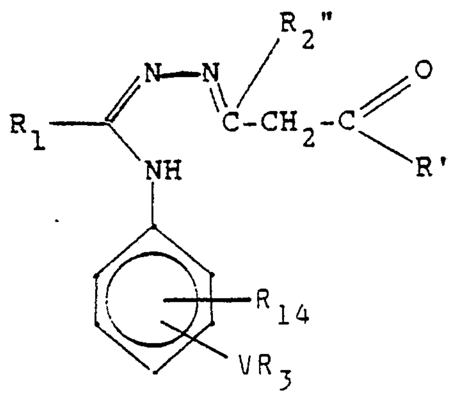

(XXII),

in der $R_1$, $R_3$, $R_{14}$ und V die obige Bedeutung haben und R′ $C_1$-$C_8$-Alkyl oder -Alkoxy und $R_2''$ $C_1$-$C_4$-Alkyl bedeutet, erhitzt

oder daß man

d) eine Verbindung der Formel

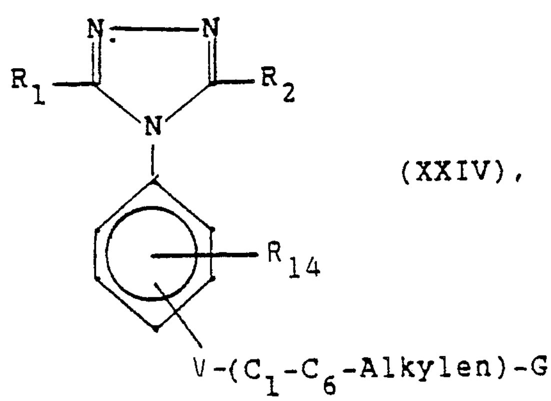

(XXIV),

in der $R_1$, $R_2$ und V die obige Bedeutung haben und G eine Abgangsgruppe "leaving group" ist, mit wolchen deprotonierten Heterocyclen $R_3$ umsetzt, die über ein Stickstoffatom mit der Alkylenkette verbunden werden sollen.

oder daß man
e) eine Verbindung der Formel XXV, in der $R_1$, $R_2$ und V die obige Bedeutung haben und M ein Alkalimetall bedeutet, mit einer Verbindung
G - $R_3$ (XXVII)
umsetzt, in der G und $R_3$ die obige Bedeutung haben,
oder daß man
f) zur Herstellung von Verbindungen der Formel I, in denen $R_3$ einen gegebenenfalls substituierten über die 4-Position verknüpften 4H-1,2,4-Triazolring bedeutet, ein Amin der Formel

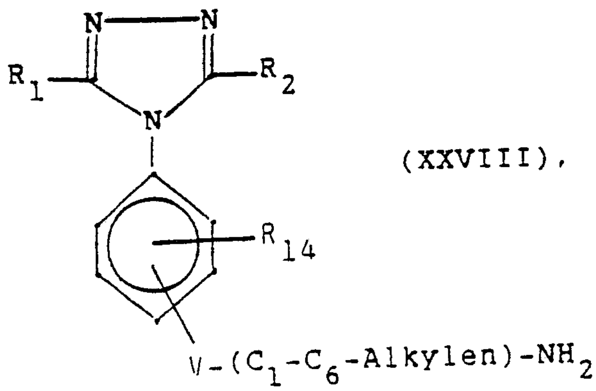

(XXVIII),

in der $R_1$, $R_2$ und V die obige Bedeutung haben, einer Ringschluß-Reaktion nach den in Verfahren 1, 2 und 3 angegebenen Methoden zur Bildung einer entsprechenden 4H-1,2,4-Triazolverbindung unterwirft, und daß man die nach den Verfahren a) bis f) erhaltenen Salze gewünschtenfalls in freie Basen, zunächst erhaltene Salze in die freien Basen überführt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 89 10 4145

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 269 938 (DELALANDE)<br>* Insgesamt *<br>-- | 1-5,7 | C 07 D 249/08<br>A 61 K 31/41<br>C 07 D 409/14<br>C 07 D 401/12<br>C 07 D 403/12<br>C 07 D 413/14<br>C 07 D 401/14<br>C 07 D 405/14 |
| Y | EP-A-0 255 028 (BOEHRINGER INGELHEIM)<br>* Insgesamt *<br>-- | 1-5,7 | |
| Y | FR-A-2 183 807 (UPJOHN)<br>* Insgesamt *<br>-- | 1-5,7 | |
| Y | FR-A-2 539 127 (ROUSSEL-UCLAF)<br>* Insgesamt *<br>----- | 1-5,7 | RECHERCHIERTE SACHGEBIETE (Int. Cl.4)<br>C 07 D 249/00<br>C 07 D 401/00<br>C 07 D 403/00<br>C 07 D 405/00<br>C 07 D 409/00<br>C 07 D 413/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7

Unvollständig recherchierte Patentansprüche:

Nicht recherchierte Patentansprüche: 6

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 20-06-1989 | ALLARD |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82